# FASCICULE DE BREVET EUROPEEN

(11) **EP 1 520 179 B1**
(45) Date de publication et mention de la délivrance du brevet: **06.01.2016**
(21) Numéro de dépôt: 03761661.2
(22) Date de dépôt: 27.06.2003
(51) Int. Cl.: C12M 1/00, C12M 1/36, G01N 35/00, C12M 1/34, C12M 3/00

(54) **PLATE-FORME ROBOTISEE DE CULTURE CELLULAIRES EN BATTERIES DE REACTEURS MINIATURISES, EQUIPE D'UN SYSTEME DE MESURE EN TEMPS REEL D'UNE PROPRIETE OPTIQUE**
PLATTFORM ROBOTERSYSTEM ZUR ZELLKULTUR MIT EINER REIHE VON MINIATURISIERTEN REAKTOREN AUSGERÜSTET MIT EINEM SYSTEM ZUR DURCHFÜHRUNG OPTISCHER MESSUNGEN IN ECHTZEIT
ROBOTIZED PLATFORM FOR CELL CULTURES IN MINIATURE REACTOR BATTERIES, EQUIPPED WITH A SYSTEM FOR REAL TIME MEASUREMENT OF CELLULAR TURBIDITY OR OTHER OPTICAL PROPERTIES

(30) Priorité: 28.06.2002 CA 2391641
(43) Date de publication de la demande: 06.04.2005
(73) Titulaire: INSTITUT PASTEUR, 75015 Paris (FR)
(72) Inventeur: BELLALOU, Jacques, F-75014 Paris (FR); FRACHON, Emmanuel, F-94220 Charenton le Pont (FR); MEIER, Alain, F-94220 Charenton le Pont (FR); LONGIN, Robert, A., F-75009 Paris (FR)
(74) Mandataire: Desaix, Anne
(86) Numéro de dépôt international: PCT/FR2003/002006
(87) Numéro de publication internationale: WO 2004/003569

(56) Documents cités:
- WO-A-93/20612
- WO-A-99/04228
- WO-A-99/27349
- US-A- 5 314 825
- US-B1- 6 307 630

## Description

### CONTEXTE DE L'INVENTION

### a) Domaine de l'invention

L'invention concerne un système automatisé de contrôle et de régulation de cultures cellulaires contenues dans une batterie de réacteurs miniaturisés qui permet, entre autres, l'injection et le prélèvement au sein des cultures.

L'invention concerne également un système et une méthode de mesure en temps réel de propriétés optiques de cultures cellulaires contenues dans une batterie de réacteurs miniaturisés. Ces propriétés optiques peuvent inclure notamment la turbidité.

### b) Brève description de l'art antérieur

Dans de nombreux laboratoires de biologie et également dans les industries de biotechnologie, il est très souvent nécessaire de cultiver des micro-organismes en milieu liquide et de mesurer la concentration des cellules à différentes étapes de la culture. Les méthodes les plus pratiques et les plus utilisées pour évaluer les concentrations cellulaires de cultures reposent sur les propriétés optiques de ces dernières.

### Systèmes connus de cultures cellulaires

Dans le domaine des technologies de cultures microbiennes, on constate qu'il existe principalement trois types de cultures : les cultures à l'échelle des laboratoires de recherche ou en recherche et développement; les cultures en fermenteurs classiques qui ont pour objectifs l'optimisation et la production; et les technologies de culture en batteries.

Les cultures à l'échelle des laboratoires sont réalisées en micro-plaques, en tubes ou en flacons agités et font appel à de faibles volumes et à du matériel d'usage simple. Elles offrent la possibilité de mener un grand nombre d'essais en parallèle. Cependant, les conditions de croissance ne sont pas optimales car des limitations nutritionnelles sont imposées aux cultures par le matériel utilisé. Il y a peu de possibilité de contrôle et de régulation des paramètres de culture, et cela génère donc de nombreuses difficultés de standardisation et de reproductibilité. Ces techniques souffrent souvent d'un certain empirisme.

Les cultures en fermenteurs classiques permettent d'assurer, dans un environnement parfaitement contrôlé par l'utilisation de capteurs, la réalisation de cultures en conditions non limitantes. Toutefois, l'équipement est sophistiqué, coûteux, et de mise en oeuvre assez lourde; le volume minimum de milieu reste élevé (0,5 litre) et ne se prête pas facilement à un grand nombre d'essais en parallèle. L'automatisation est limitée et ne s'applique qu'au seul réacteur en culture. Ce type d'appareillage nécessite, le plus souvent, un expérimentateur formé à ces technologies. Les systèmes de lecture optique sont coûteux et encombrants.

Les technologies de culture en batterie, telle que commercialisés par les sociétés comme DASGIP [Modèles Unitron-pro^{MC} (16 x 500 mL) ou Stimmer-pro^{MC} (8, 12, 16 x 150 à 300 mL)] ou INFORS [Modèle Profors^{MC} (jusqu'à 16 x 150 mL)], proposent un appareillage qui contrôle jusqu'à 16 cultures en flacons ou mini-réacteurs aérés, avec mesure et régulation du pH, de la pO₂ et de la température. La vocation de ces appareillages est principalement de pouvoir réaliser, dans des conditions standardisées, des cultures en parallèle de façon aisée et apporte une réponse aux limitations nutritionnelles rencontrées pour des cultures en flacons agités mal aérés. Cependant ces dispositifs ne disposent pas de mesure de la turbidité en ligne, et de ce fait ne sont pas adaptés à un fonctionnement totalement automatisé basé sur la mesure directe de ce paramètre important.

Les démarches expérimentales les plus souvent mises en oeuvre dans le domaine de la bactériologie industrielle ou bio-médicale, ont en commun les objectifs suivants :
- Cultiver de nombreuses souches de micro-organismes dans le but de comparer leurs performances puis de sélectionner la souche la plus adaptée au procédé étudié;
- Produire, à des niveaux élevés, des molécules comme les protéines recombinantes; et
- Transférer les résultats d'optimisation des paramètres de culture les plus importants d'un procédé donné, d'une échelle de laboratoire à une échelle de production pilote.

Ces différentes démarches rencontrent en général les mêmes difficultés techniques, car aucune des technologies existantes, présentant chacune des intérêts respectifs, ne peut répondre totalement aux exigences et contraintes qui existent présentement. Ces exigences naissent de la très grande diversité des souches à cultiver (exigences nutritionnelles différentes, température, etc.) et des séquences de gènes hétérologues à exprimer dans ces souches.

Il existe donc un besoin pour un système miniaturisé et automatisé de cultures cellulaires qui puisse répondre à l'ensemble de ces exigences.

La présente invention répond aux besoins identifiés ci-dessus et à d'autres besoins comme cela sera apparent à une personne versée dans le domaine à la lecture de la présente description de l'invention.

### Systèmes connus servant à mesurer la turbidité

Parmi les turbidimètres existant, on distingue les appareils de mesure en discontinu ou manuel, avec lesquels on mesure de manière répétée la lumière transmise et/ou réfléchie par un milieu liquide, et les appareils de mesure en ligne, pour lesquels des sondes de mesure de turbidité sont introduites à l'intérieur d'un milieu liquide et reliées à un système d'enregistrement.

Les appareils de mesure en discontinu, tels que néphélomètres, colorimètres, spectrophotomètres et turbidimètres mixtes, ne permettent pas, pour la plupart d'entre eux, de mesurer des turbidités élevées. Certains turbidimètres mixtes ont été conçus pour couvrir une plus large gamme de turbidité, mais il s'agit d'appareils coûteux.

Les turbidimètres en ligne, dont les gammes de mesure peuvent être assez vastes, ont pour inconvénients de nécessiter des volumes de culture importants (à partir de 250 mL) à cause de l'encombrement des sondes introduites à l'intérieur des cultures, ce qui limite leur utilisation au sein de fermenteurs miniaturisés. De plus, les cultures en fermenteurs sont généralement aérées et agitées, ce qui provoque la formation de nombreuses bulles perturbant fortement les mesures de turbidité. Ces dispositifs sont aussi pénalisés par leur coût élevé.

D'autres turbidimètres de mesure en direct existant sont coûteux et difficiles à utiliser car leurs capteurs ont besoin d'être d'autoclavés, souffrent d'encrassement, et offrent une gamme de lecture moins étendue. Ces turbidimètres nécessitent, pour chaque culture, différents capteurs insérés dans le réacteur et ceux-ci sont sujets à des dérives en cours de fonctionnement.

Les autres systèmes existants sont fondés sur des estimations indirectes de la densité bactérienne, à travers la mesure de divers paramètres de culture (pH, consommation d'oxygène, potentiel rédox, concentrations de substrats, etc.).

La demande de brevet internationale WO 99/27349 (GAILLON et al.) divulgue un capteur de mesure en continu de la turbidité des cultures. Cependant ce dispositif est statique et chaque réacteur doit être équipé de son propre capteur constitué d'un couple de diodes particulier (paire de diodes émettrice / réceptrice). En raison de la différence de sensibilités et de performances des diodes les unes par rapport aux autres, il s'est avéré difficile de calibrer correctement ces capteurs. En effet, il est difficile d'obtenir, par les différents capteurs, la même réponse pour une suspension de turbidité donnée. La demande WO99/04228 décrit un instrument pour l'analyse optique qui utilise des sources et détecteurs placés au-dessus et en dessous des échantillons, dépourvu de système de régulation de température. Le brevet US 6,307,630 décrit un turbidimètre pour suivre la turbidité d'échantillons d'eau, la turbidité étant mesurée en lumière dispersée. Le brevet US 5,314,825 décrit un analyseur chimique pourvu d'un capteur fixe et d'un porte-échantillon mobile.

Actuellement, il n'existe aucun système automatisé et mobile, peu encombrant, peu coûteux et capable de mesurer, avec précision, la turbidité de cultures microbiennes réalisées en tubes ou en réacteurs agités, sur une large gamme de valeurs.

Il existe donc un besoin pour un système de mesure de propriétés optiques de cultures cellulaires, n'ayant pas les inconvénients des appareils connus de l'art antérieur, qui soit peu encombrant, d'un coût raisonnable et capable de mesurer avec précision, et en temps réel, la turbidité de cultures microbiennes réalisées en parallèle sur une large gamme de valeurs.

### RÉSUMÉ DE L'INVENTION

Un objectif de la présente invention est de fournir une plate-forme de cultures cellulaires et une méthode de mesure de propriétés optiques de cultures cellulaires, n'ayant pas les inconvénients des appareils connus de l'art antérieur.

En particulier, un objet selon un aspect préférentiel de la présente invention est de fournir un système peu encombrant, d'un coût raisonnable et capable de mesurer précisément et en temps réel la turbidité de plusieurs cultures cellulaires réalisées simultanément sur une large gamme de valeurs.

Un des objectifs de la présente invention est de fournir une plate-forme miniaturisée et automatisée de cultures cellulaires menées en parallèle et ayant les mêmes performances que les fermenteurs de laboratoire.

La présente invention concerne donc une plate-forme automatisée et robotisée comportant une batterie de réacteurs miniaturisés dont le volume utile est compris entre 2 mL et 500 mL contenant chacun une culture cellulaire, la plate-forme comprenant :
un capteur externe pour mesurer au moins une propriété optique de chaque culture cellulaire contenue dans chaque réacteur miniaturisé ;
un porte-capteur mobile en forme de fourche ou d'étrier apte à recevoir au moins un capteur externe, le porte-capteur mobile comportant des moyens de déplacement du capteur pour déplacer le capteur externe d'un réacteur miniaturisé à un autre et pour permettre la mesure en temps réel de ladite au moins une propriété optique ;
des moyens de contrôle et de traitement pour recevoir en temps réel des mesures de la propriété optique du ou des capteurs externes et pour contrôler en temps réel un déplacement du porte-capteur mobile ; et
un système de régulation de la température pour chaque réacteur.

L'invention concerne également une méthode pour mesurer automatiquement au moins une propriété optique de cultures cellulaires contenues dans une batterie de réacteurs miniaturisés dont le volume utile est compris entre 2 mL et 500 mL, comprenant les étapes de :
mesure automatique d'au moins une propriété optique d'une culture contenue dans l'un des réacteurs miniaturisés à l'aide d'un capteur externe ;
déplacement robotisé du capteur externe vers un autre réacteur miniaturisé ; et
mesure automatique d'au moins une propriété optique d'une culture contenue dans un autre réacteur miniaturisé à l'aide du capteur externe.

### DÉFINITIONS

Par réacteurs miniaturisés on entend un fermenteur dont le volume utile de culture est compris entre au moins 2 mL et au plus 500 mL. Plus spécifiquement, on entend par mini-réacteurs, les fermenteurs dont le volume utile de culture est compris entre 60 mL et 500 mL, et par micro-réacteurs on entend les fermenteurs dont le volume utile est compris entre 2 mL et 60 mL.

Par culture cellulaire on entend une culture de micro-organismes tel que les bactéries, les levures, les champignons et toutes autres cellules eucaryotes.

Par Vref on entend la tension nominale fixée aux bornes de la diode réceptrice du capteur de turbidité, la valeur de ce paramètre détermine la sensibilité de la mesure Ve.

Par Ve on entend la tension mesurée aux bornes de la diode émettrice du capteur de turbidité, cette valeur est fonction de la turbidité de la culture.

Par DO on entend la densité optique mesurée par un spectromètre.

### BRÈVE DESCRIPTION DES DESSINS

La **Figure 1** est une vue de face d'une plate-forme de cultures cellulaires comportant une batterie de micro-fermenteurs, des systèmes de régulation de la température par effet Peltier, un capteur externe monté sur un porte-capteur mobile, et des modules électroniques d'acquisition et de contrôle, d'après une réalisation préférentielle de la présente invention.
Les **Figures 2A, 2B** et **2C** sont respectivement des vues schématiques en coupe, de dessus et de côté d'un porte-capteur mobile, d'après une réalisation préférentielle de la présente invention.
Les **Figures 2D, 2E, 2F** et **2G** sont respectivement une vue de dos, de côté et de face d'une diode émettrice, ainsi qu'une vue de dos d'une diode réceptrice servant à mesurer la turbidité, d'après une réalisation préférentielle de la présente invention.
La **Figure 3** est une vue de côté d'un porte-capteur en forme de fourche destiné à être monté sur un système de guidage d'une plate-forme de cultures cellulaires, d'après une réalisation préférentielle de la présente invention.
La **Figure 4** est une vue en perspective d'un porte-capteur monté sur deux rails d'une plate-forme de cultures de micro-organismes, d'après une réalisation préférentielle de la présente invention.
La **Figure 5** est une vue de face du porte-capteur montré à la Figure 4.
La **Figure 6** est une vue de côté d'un élément du capteur de turbidité détaillant le support pour installation sur le porte-capteur montré à la Figure 4, d'après une réalisation préférentielle de la présente invention.
La **Figure 7** est une vue de dessus d'une batterie de mini-réacteurs de 250 mL équipée d'un bras mobile assurant un mouvement circulaire, pour mesurer la turbidité dans les réacteurs, d'après une réalisation préférentielle de la présente invention.
La **Figure 8** est une vue de face d'une plate-forme de cultures cellulaires munie de blocs de régulation thermique par effet Peltier, d'après une réalisation préférentielle de la présente invention.
Les **Figure 9A** et **9B** sont respectivement des vues de face et de côté d'un micro-fermenteur inséré dans son support thermostaté de type Peltier, d'après une réalisation préférentielle de la présente invention.
La **Figure 10** est une vue de plan d'éléments composant un support du réacteur, thermostaté de type Peltier, d'après une réalisation préférentielle de la présente invention.
La **Figure 11** montre la courbe reliant l'évolution de la mesure de la turbidité à la mesure manuelle de la DO pour des cultures menées en parallèle avec 7 micro-fermenteurs et une souche de *E. coli.*
La **Figure 12** montre une interface graphique d'un programme de contrôle du système selon l'invention, montrant entres autres des courbes d'évolution de la turbidité en fonction du temps dans chacun des micro-fermenteurs.

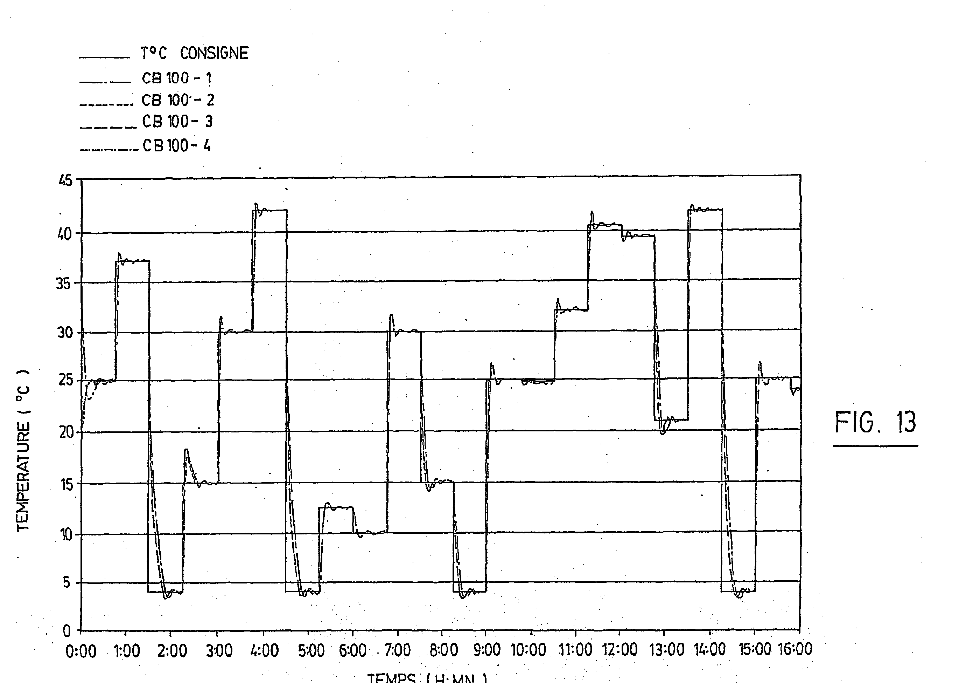

La **Figure 13** montre des courbes de température en fonction du point de consigne et du temps pour quatre régulateurs thermiques, d'après une réalisation préférentielle de la présente invention.
Les **Figures 14A, 14B** et **14C** sont respectivement des vues en coupe d'un système d'injection et de prélèvement dans des positions haute et basse, ainsi qu'une vue de face d'un piston selon la présente invention.
Les **Figures 14D****,** **14E, 14F, 14G** et **14H** sont des vues en coupe d'un purgeur dans diverses positions selon la présente invention.

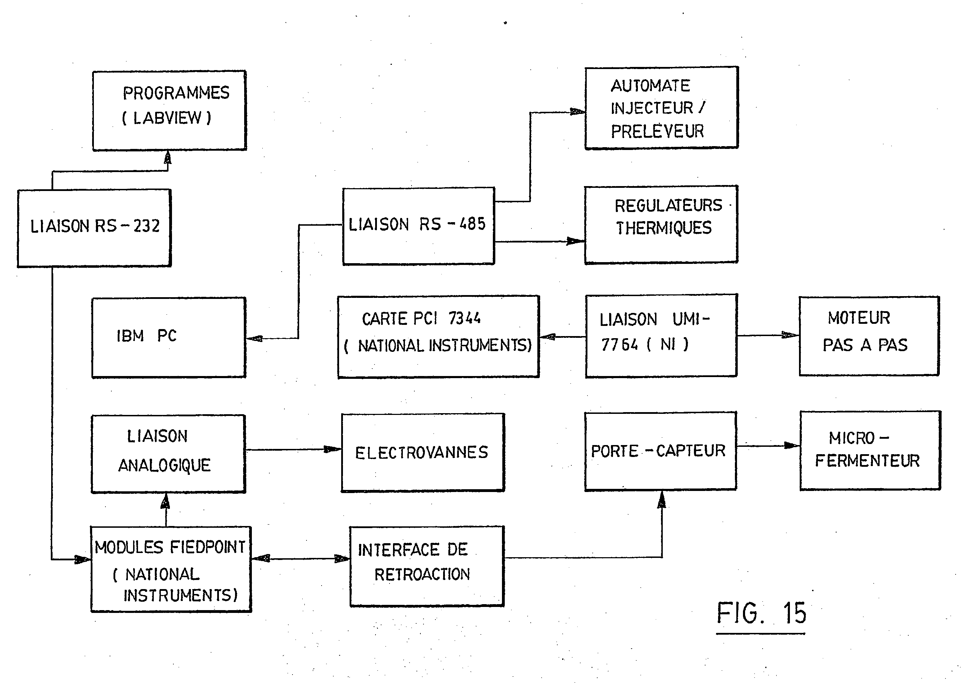

La **Figure 15** montre un schéma de fonctionnement d'une batterie de micro-fermenteurs selon la présente invention.

### DESCRIPTION DÉTAILLÉE DE L'INVENTION

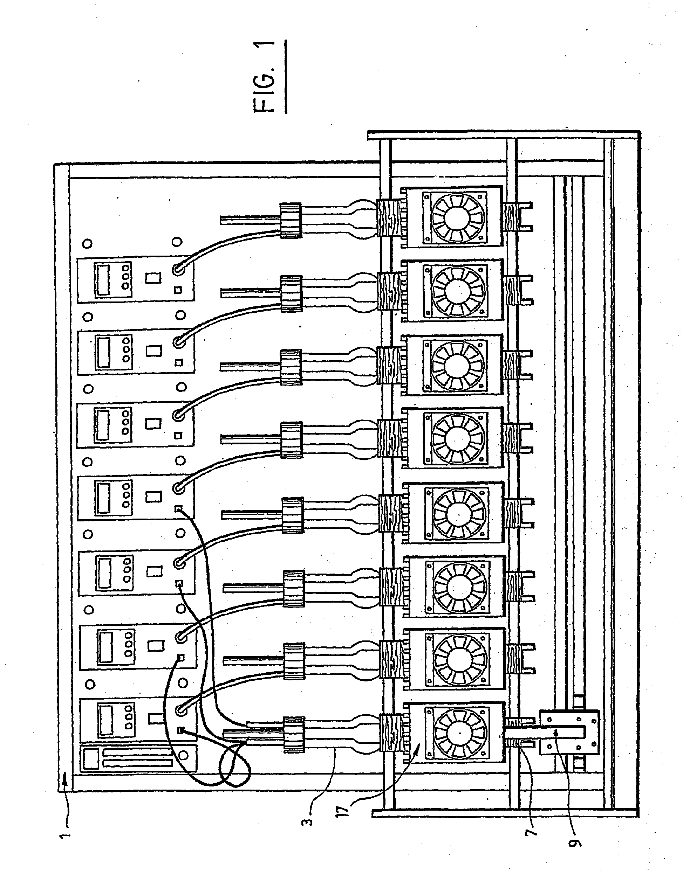

La Figure 1 montre une plate-forme 1 de cultures cellulaires d'après une réalisation préférentielle de la présente invention. La plate-forme 1 repose sur le concept original de la miniaturisation et de l'automatisation de cultures microbiennes, de faible volume (60 mL), réalisées en micro-fermenteurs 3. De préférence, la plate-forme 1 comporte une batterie de huit micro-fermenteurs 3, mais ce nombre peut varier selon les applications ou les besoins, de même que le volume de chaque micro-fermenteur 3.

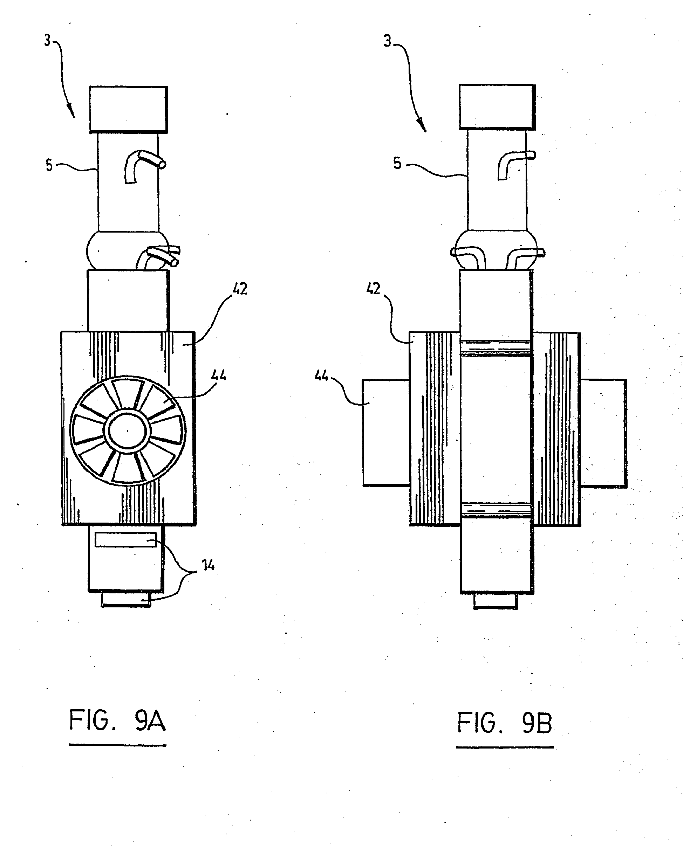

Le corps des micro-fermenteurs 3 peut être constitué de réacteurs fait en tubes de verre 5 (voir les Figure 9A et 9B) de section carrée de 2 cm, de 16 à 18 cm de hauteur, munis de tubulures reliées à des tuyaux permettant la circulation des fluides liquides et gazeux. Les tubes de verre 5 doivent être normalement stérilisés avant chaque nouvel usage. Ces tubes de verres peuvent également être remplacés par des réacteurs stériles en matière plastique, par exemple en polycarbonate, prêts à l'emploi et à usage unique.

Les micro-fermenteurs 3 sont assemblés en batteries au sein d'un système compact et ergonomique avec supervision intégrée de la conduite des cultures. Tel qu'il sera expliqué plus bas, de par leur niveau d'équipement en capteurs et leur degré d'automatisation, ces micro-fermenteurs 3 offrent les mêmes potentialités que des fermenteurs de laboratoire de plus grand volume.

### Un ou plusieurs capteurs montés dans un porte-capteur mobile

Dans le système de batterie de première génération connu de l'art antérieur, chaque micro-fermenteur est placé dans un bloc métallique dans lequel est insérée une paire de diodes émettrice et réceptrice. Ces diodes donnent satisfaction quant aux performances (gammes étendues de turbidité, implantation à l'extérieur des fermenteurs, faible coût, etc.). Cependant, chaque bloc de culture est équipé de son propre capteur possédant une paire de diodes particulière. Il s'est avéré difficile de calibrer correctement ces capteurs les uns par rapport aux autres. Par exemple, sur 100 diodes LED ou récepteurs Darlington, seuls 10% se révélaient utilisables en présentant une dynamique de réponse comparable. De plus, il s'est avéré que l'appariement des couples de diodes LED et Darlington pour chaque capteur se traduisait par une dynamique de réponse voisine mais non identique. L'utilisation d'un potentiomètre de réglage de gain permettait d'étalonner les différents couples pour une DO donnée (par exemple 1) mais non sur l'ensemble de la gamme de mesure (par exemple de DO 0,5 à DO 100 pour une culture Haute Densité Optique HDO). Ainsi, les courbes de réponse des différents capteurs en fonction de la turbidité (DO) d'une culture ou d'une suspension cellulaire n'étaient pas superposables. Aussi, pour convertir automatiquement les valeurs mesurées de Ve et les afficher en unités de DO, il aurait fallu introduire dans les programmes autant d'équations de modélisation reliant le Ve à la DO que de capteurs utilisés, ce qui n'était pas acceptable.

La plate-forme 1 selon la présente invention apporte une solution à ce problème en se basant sur le concept original d'un seul et même capteur externe et mobile 7 servant à la mesure séquentielle d'une propriété optique d'une batterie de cultures cellulaires réalisées en micro-fermenteurs 3, à l'aide d'un porte-capteur mobile 9 entraîné par un moteur 8 pas à pas, sous le contrôle d'un programme informatique.

En particulier, le capteur 7 permet une mesure directe et en ligne de la turbidité de l'ensemble des cultures en cours dans les divers micro-fermenteurs 3. Le capteur 7 de mesure de turbidité est monté dans un porte-capteur mobile 9 qui se déplace à l'aide d'un moteur sur un axe linéaire le long d'une rangée de micro-fermenteurs 3 et permet de mesurer séquentiellement, avec précision et reproductibilité, la turbidité dans chacun des micro-fermenteurs 3 sans intervention de l'opérateur sur les cultures en cours.

Le positionnement du porte-capteur 9 à l'aide du moteur pas à pas peut se faire avec une résolution de l'ordre 10 à 100 µm environ selon le mécanisme de déplacement utilisé, avec une très bonne fiabilité. Il est donc possible de faire plusieurs lectures successivement sur un même micro-fermenteur 3. Il est à noter qu'au lieu d'un moteur pas à pas, des variantes consistent à utiliser un bras circulaire (servo-moteur) ou un bras de robot ou tout autre système qui permet le déplacement relatif entre le capteur 7 et les micro-fermenteurs 3.

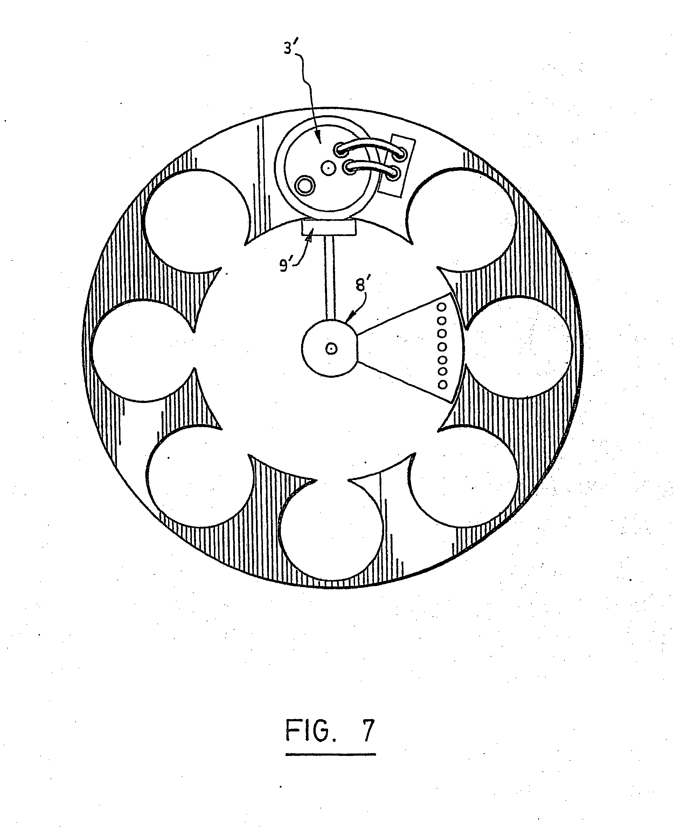

En référence à la Figure 7, on montre une variante particulière de porte-capteur mobile 9' relié à un moteur 8' qui lui permet de défiler selon un mouvement circulaire devant des mini-fermenteurs 3' où se déroulent des cultures cellulaires.

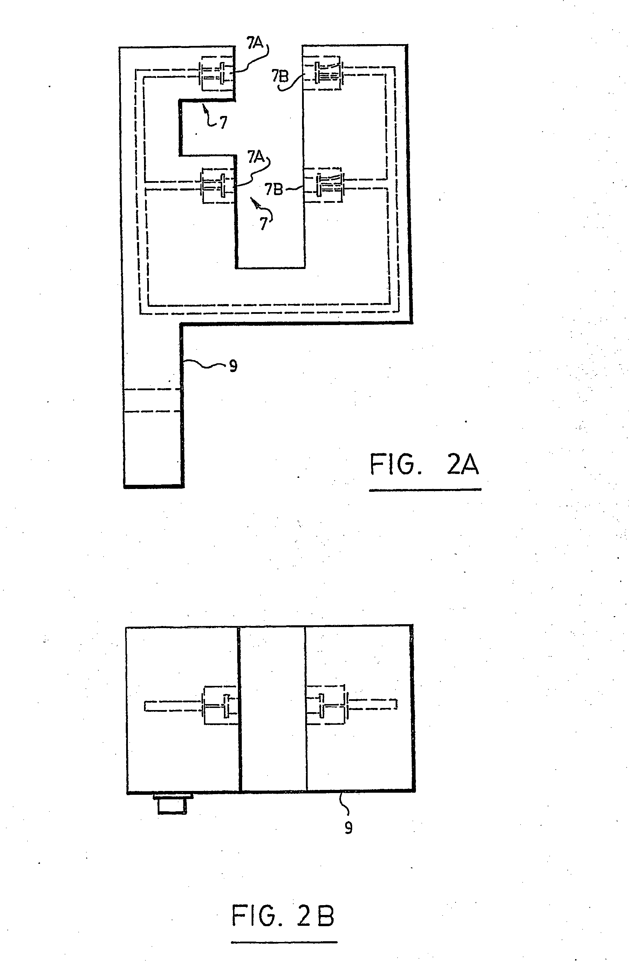

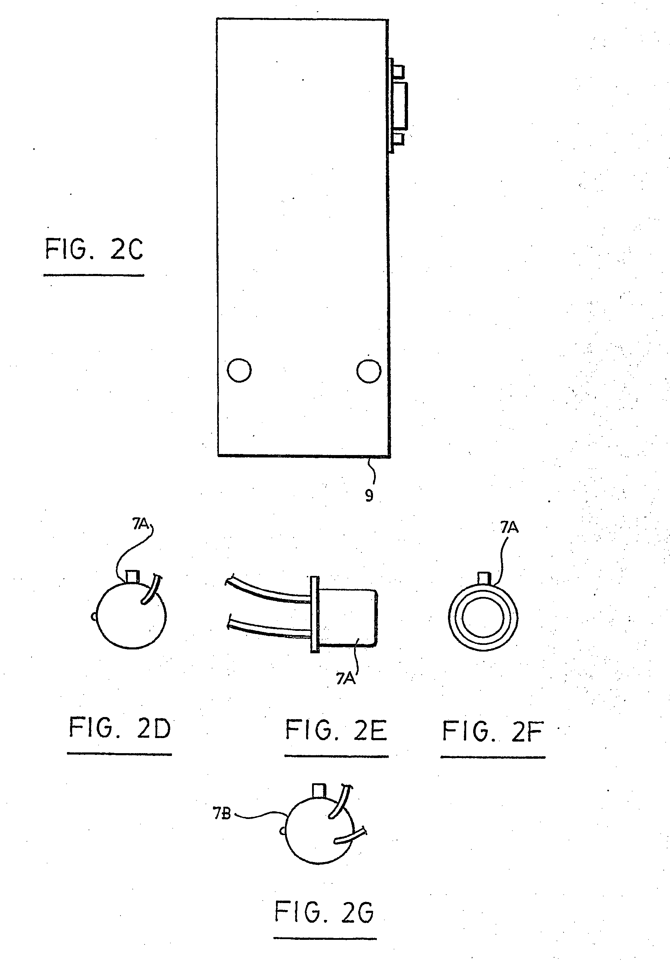

De préférence, tel que montré à la Figure 2A, le capteur de turbidité 7 se compose d'au moins un couple de composants optiques : une diode émettrice 7a de lumière infrarouge et un Photodarlington récepteur 7b de la lumière résiduelle transmise qui a traversé le micro-fermenteur 3. On montre également que plus d'un capteur 7 peut être monté sur le porte-capteur 9. L'insertion de deux couples de diodes 7 offre la possibilité de réaliser une mesure à des hauteurs différentes du réacteur de culture.

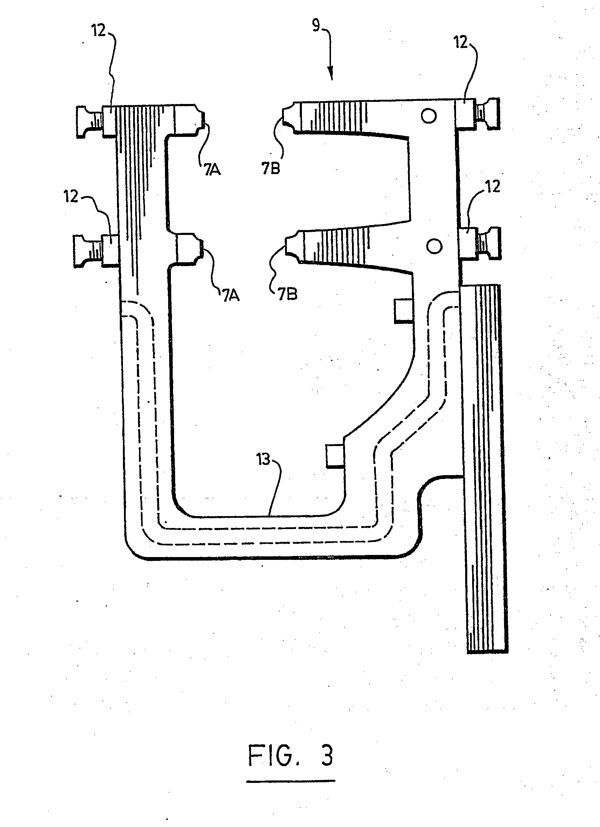

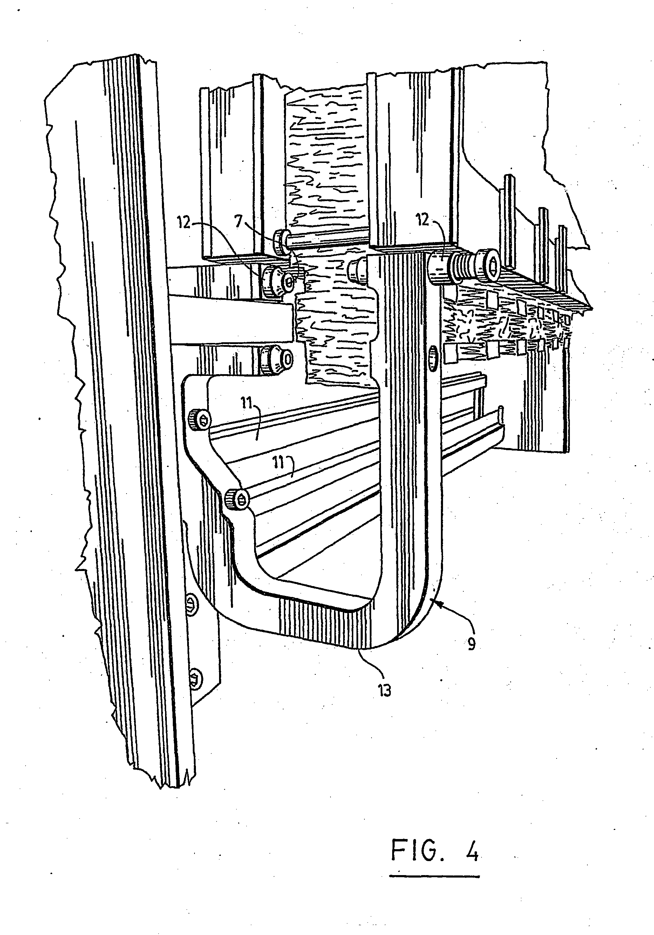

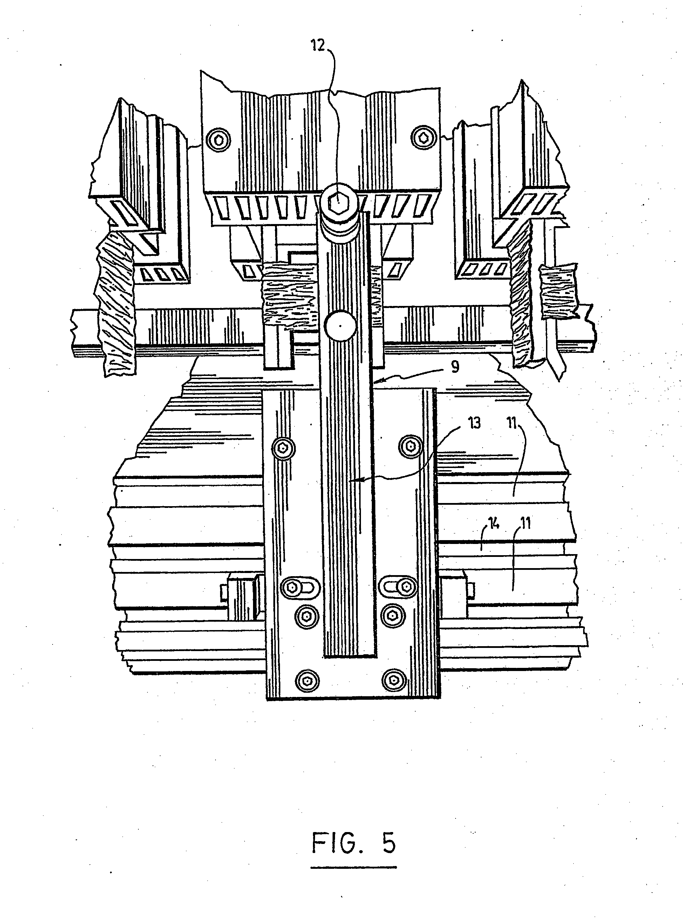

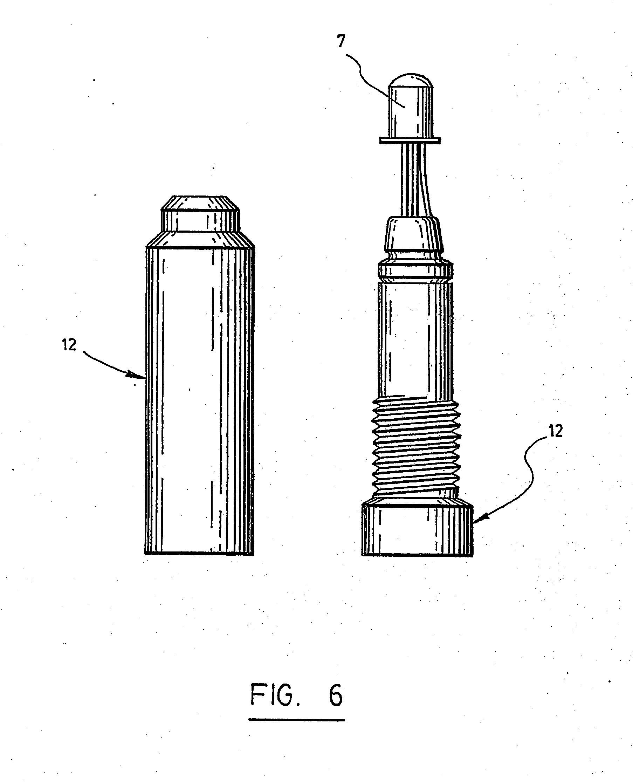

Tel qu'illustré aux Figures 3 à 5, les couples de diodes 7 peuvent être fixés sur un porte-capteur 9 en forme de fourche solidaire 13 ayant deux chariots mobiles qui se déplacent le long de deux rails 11 sous l'action d'une courroie crantée entraînée par un moteur pas à pas. Dans ce cas, le porte-capteur 9 est muni de supports 12 de diodes amovibles (voir aussi à la Figure 6), facilitant l'insertion et le changement des diodes 7, selon les besoins. Le fait de disposer d'un porte-capteur 9 en forme de fourche ou étrier dont on peut faire varier la forme et les côtés permet d'y fixer différents capteurs disponibles sur le marché ou nouveaux, et ainsi d'augmenter les possibilités d'analyse des cultures microbiennes, par exemple, en utilisant plusieurs capteurs de turbidité à plusieurs hauteurs. Dans d'autres cas, ce dispositif peut ouvrir le champ à des études de décantation ou de floculation de souches microbiennes ou d'adhérence dans le réacteur, ou pour mettre au point des procédés, ou pour des études d'évolution ou d'enrichissement de souches, à l'aide de cultures cycliques.

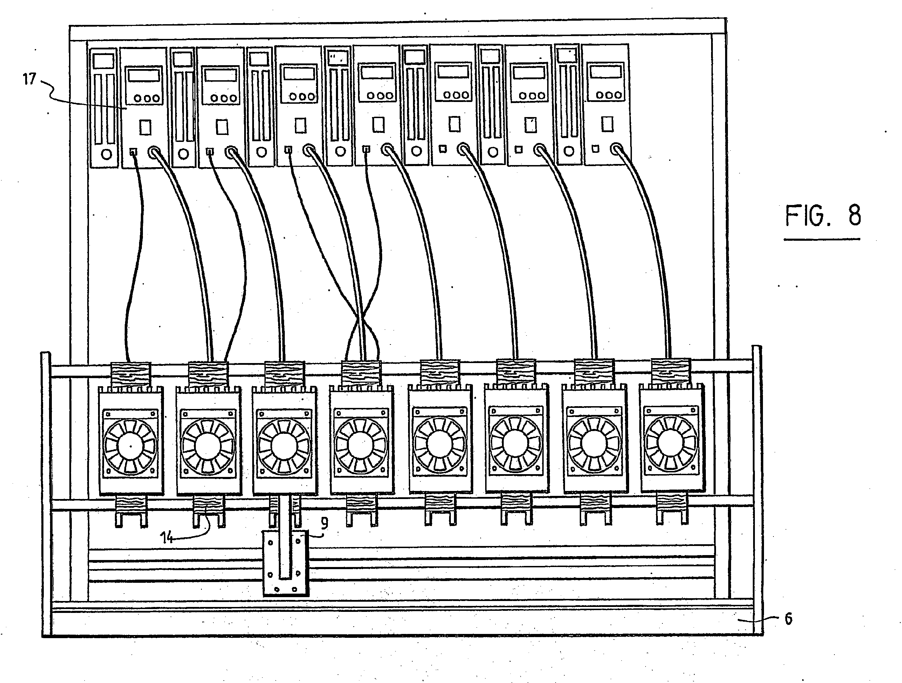

Le porte-capteur 9 est préférablement muni de deux glissières ou guides qui sont déportées vers l'arrière afin de protéger les parties mobiles des projections ou des fuites éventuelles. Un bac de rétention 6 (voir à la Figure 8) peut être disposé sous la batterie en cas d'un écoulement de culture, dans le cas d'un bris d'un micro-fermenteur 3 par exemple. Le mode de guidage par rails parallèles élimine une grande partie des vibrations et permet de réduire la distance entre les composants optiques et les parois des micro-fermenteurs 3, réduisant la dispersion du faisceau lumineux. Une fenêtre de mesure 14 située dans le bloc support permet une mesure optique à mi-hauteur du micro-fermenteur 3. Le bas du micro-fermenteur 3 est également accessible pour un deuxième capteur. Différents modèles de micro-fermenteurs peuvent être utilisés en fonction du type d'application.

Le porte-capteur 9 peut également être adapté pour accepter des fibres optiques ou d'autres types de capteurs (bioluminescence - e.g. pour la production de protéines recombinantes- , phosphorescence, colorimétrie, fluorescence, etc.).

En particulier, un capteur de lumière (photomultiplicateur (PM)) peut également être utilisé pour l'étude de l'expression de gènes rapporteurs comme le gène *lux.* Ce capteur de luminescence peut être monté sur le même porte-capteur mobile 9, déjà équipé du capteur de mesure de la turbidité 7.

Il est à noter que ces capteurs de lumière sont très coûteux (1000 euros pour le seul PM) et nécessitent un étalonnage complexe. D'après l'ancien concept, il aurait fallu installer un capteur au niveau de chaque micro-fermenteur. Par contre, l'utilisation d'un seul porte-capteur mobile qui défile devant les micro-fermenteurs réduit, par exemple, de 8 fois les coûts d'équipement d'une batterie de 8 micro-fermenteurs.

Bien entendu, différentes géométries du porte-capteur mobile 9 peuvent aussi être envisagées. La modification du porte-capteur 9 permet d'adapter le système de mesure à la géométrie du réacteur, selon les besoins, que ce soit des tubes de plusieurs micro-fermenteurs de 60 mL, ou bien des tubes de 2 à 5 mL ou encore des mini-réacteurs de 250 à 500 mL. De même, il est possible de choisir avec précision les angles et distances de lecture.

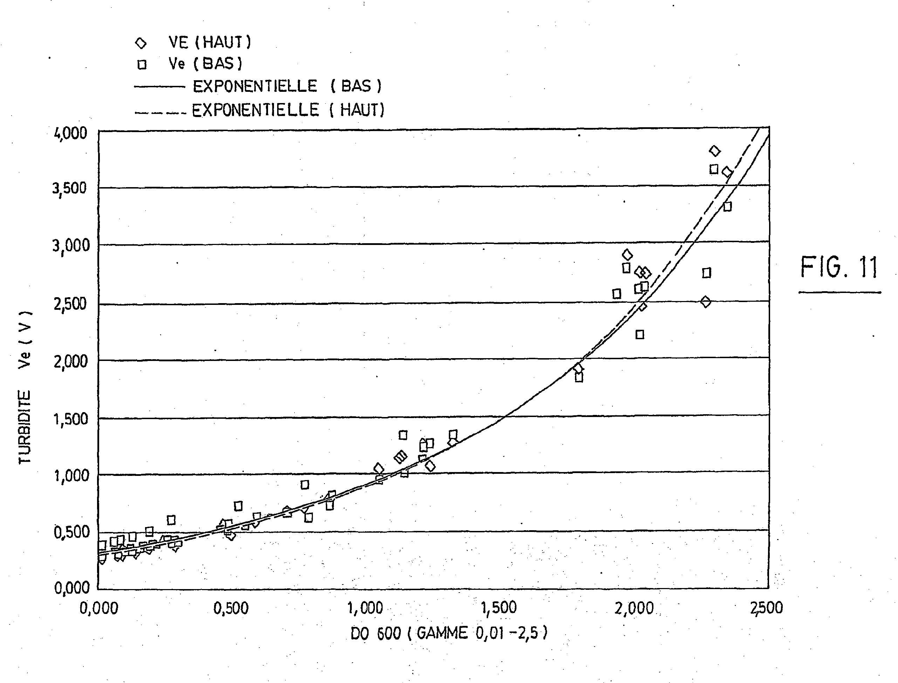

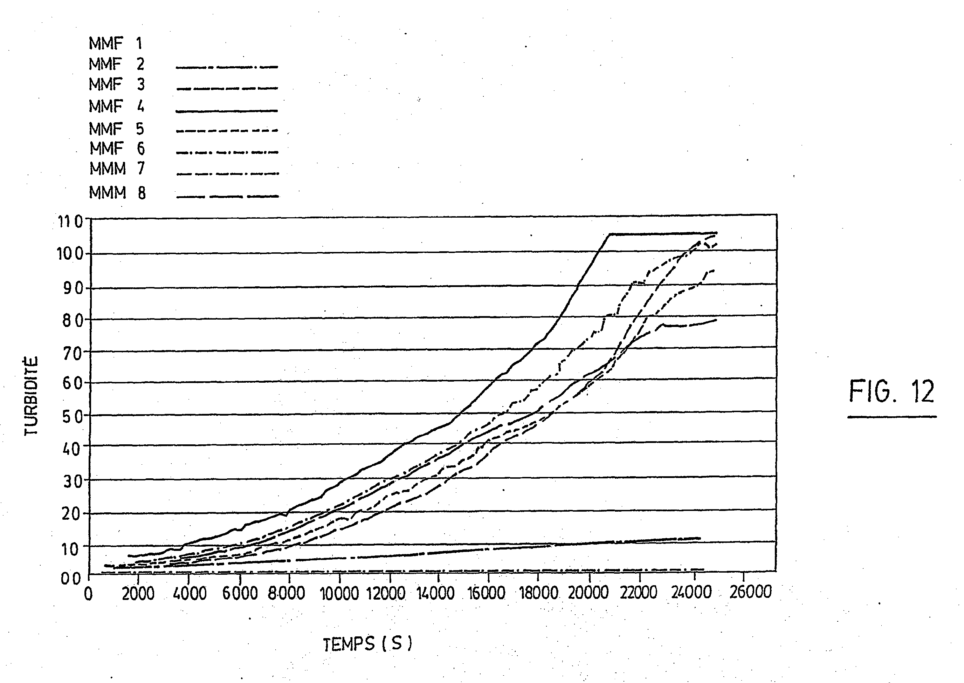

En référence à la Figure 11, on montre l'évolution de la tension Ve mesurée aux bornes de la diode émettrice 7a en fonction de la turbidité d'une culture. En référence à la Figure 12, on montre l'évolution de la tension Ve aux bornes de la diode émettrice 7a en fonction du temps. On peut ainsi déterminer la concentration cellulaire d'une culture dans chaque micro-fermenteur 3 (concentration exprimée en unités de DO par exemple). L'équation reliant Ve à la DO, introduite dans le programme de contrôle, permet d'avoir en ligne la valeur de celle-ci. Ce dispositif permet une mesure de la turbidité dans différentes gammes de sensibilité, correspondant à des DO de 0,05 à plus de 100 unités de DO (pour la bactérie modèle *Escherichia coli*) et de 0,05 à plus de 300 pour la levure modèle *Saccharomyces cerevisiae.* Il permet de travailler avec des milieux minimaux ou complexes.

Les séquences de déplacement du porte-capteur 9 et de mesure de la turbidité sont supervisées par le programme informatique. En fonction de la sensibilité choisie (valeur du Vref) pour la mesure à effectuer, il est possible de mesurer des turbidités correspondant à une gamme de densités optiques comprises entre 0,05 et 100 (pour *E*. *coli*).

Les séquences de déplacement du porte-capteur 9 et de lecture de la turbidité constituent la boucle principale du programme informatique utilisé (dépôt Institut Pasteur 2002). Ce dernier se base sur un fichier de configuration décrivant le type de matériel utilisé et le mode de fonctionnement. Ainsi, le programme informatique peut s'adapter à diverses solutions de motorisation. Pour déplacer le porte-capteur 9, on a sélectionné un moteur 8 pas à pas (SANYO DENKI type 103H71260440) fonctionnant en boucle ouverte et en mode micro-step (1/8^{ème} de pas pour une résolution de 200 pas/tour). La carte de contrôle (PCI-7344, National Instruments) installée dans l'ordinateur peut piloter quatre systèmes d'axes indépendants ou coordonnés. Un boîtier de connexion (UMI-7764, National Instruments) relie la carte de commande moteur à la carte de puissance (SANYO DENKI). Pour un fonctionnement silencieux et rapide, une transmission par courroie crantée a été privilégiée. Deux interrupteurs délimitent la zone de déplacement (650 mm pour 8 micro-fermenteurs). La résolution linéaire finale est de 80 micromètres pour une vitesse du chariot de 42,6 cm/s. Sous contrôle du logiciel informatique, le module porte-capteur 9 se positionne séquentiellement en face de chaque micro-fermenteur 3 actif pendant la durée nécessaire aux diverses opérations de mesure. La fréquence de cette boucle ainsi que le délai entre deux déplacements sont définis par l'utilisateur.

Comme tout appareillage optique, il est nécessaire de contrôler périodiquement la réponse du capteur 7 en fonction de la turbidité. Pour cela, un ou plusieurs tubes étalons, contenant des produits turbides (comme la formazine), des suspensions cellulaires ou des substances opaques, de turbidité connue, sont disposés dans des emplacements appropriés de la batterie. Au début d'une expérimentation ou en cours de manipulation, le programme réalise automatiquement la lecture successive des ces tubes, pour un calibrage du capteur : soit en unités NTU (Nephelometric Turbidity Units), soit en unités de Densité Optique (DO), soit en unités d'opacité relatives. Ainsi, l'étalonnage des capteurs 7 est effectué automatiquement et élimine toute incertitude sur la stabilité et les dérives possibles, problème particulièrement gênant dans le cas de comparaison de souches ou de procédés.

La plate-forme 1 selon l'invention permet de garantir la standardisation des cultures car le présent procédé est le seul à proposer une mesure directe, séquentielle et en ligne de la turbidité de toutes les cultures en cours par une cellule unique de mesure à défilement. Le système permet également de connaître et de comparer en temps réel la phase de croissance de toutes les cultures réalisées en parallèle.

Le système selon l'invention permet de réaliser des cultures dans des réacteurs de volumes variables en bénéficiant des mêmes fonctionnalités d'automatisation et de standardisation que les cultures en tubes de 60 mL, par exemple par l'apport différentiel d'O₂ et l'introduction de l'inducteur à la DO programmée.

En particulier, le présent système permet les cultures automatisées de très faibles volumes pour la sélection de nouvelles molécules à usage thérapeutique, dirigées contre des souches bactériennes maintenues en croissance optimale ou dans des conditions prédéfinies par l'expérimentateur. Ces cultures sont réalisées dans des tubes aérés (2 à 5 mL), portés par des racks, avec mesure en ligne de la turbidité par le capteur à défilement et injection programmée de toute molécule en cours de culture.

Le présent système permet également les cultures automatisées de mini réacteurs cylindriques de (250 à 500 mL) qui présentent à une hauteur convenable une excroissance avec deux faces parallèles et deux faces oblongues permettant l'écoulement aisé du milieu de culture entraîné par un système d'agitation. Le capteur à défilement passe successivement devant les excroissances des mini-fermenteurs de la batterie et se positionne au niveau des deux faces parallèles pour une lecture convenable. Dans la démarche d'études systématiques des protéines codées par les gènes de génomes séquencés, de nombreuses protéines ne s'expriment que très faiblement. Ces protéines sont toxiques pour les souches bactériennes qui hébergent les gènes correspondants, ou présentent des caractéristiques biochimiques et fonctionnelles (protéines membranaires) peu favorables à leur expression sous forme soluble. L'augmentation des volumes de culture, couplée aux avantages de l'automatisation, devrait permettre de compenser ces faibles niveaux d'expression et de répondre ainsi aux attentes des études de fonctionnalité de ces protéines. Cette technologie pourra également s'appliquer à l'optimisation de procédés devant être transférés à des échelles importantes de production.

La plate-forme 1 selon la présente invention évite l'implantation de capteurs encombrants à l'intérieur des réacteurs. La localisation à l'extérieur du réacteur d'un ou plusieurs capteurs permet de réduire la taille du réacteur sans diminuer le volume utile. Cela permet d'obtenir des batteries plus petites pouvant s'implanter facilement dans les laboratoires. L'utilisation de ces batteries est également compatible à l'intérieur d'un Poste de Sécurité Microbiologique (PSM).

Un autre des avantages reliés au système selon la présente invention est que le fait de disposer, à tout moment, de la turbidité des cultures ainsi que de l'historique de son évolution, permet de déclencher des événements liés à la quantité de cellules et au taux de croissance, comme, par exemple, des ajouts de milieux de culture ou des solutions d'inducteur (via l'automate injecteur/échantillonneur qui sera détaillé plus bas). De même, la concentration d'O₂ peut être accrue, la température modifiée, des prélèvements effectués. D'une façon similaire, le pH peut être régulé. La présente plate-forme permet donc une augmentation très importante des possibilités de contrôle des cultures et des performances d'analyse.

La plate-forme 1 selon la présente invention permet une diminution des coûts, tout en intégrant les meilleures optiques pour l'amélioration des capteurs. Elle permet l'intégration d'une optique particulière (fibres optiques, filtres, spectres, capteur à forte puissance, etc.) et le coût est divisé par plus de n fois le nombre de réacteurs utilisés.

De plus, les capteurs 7 étant extérieurs au tube de culture ne nécessitent pas de stérilisation, celle-ci étant une contrainte supplémentaire pour les capteurs internes existants.

L'utilisation d'un capteur mobile selon la présente invention résout donc le problème majeur de l'invention divulguée dans la demande WO 99/27349, à savoir la difficulté d'étalonnage de plusieurs capteurs statiques qui sont dédiés à leurs propres micro-fermenteurs. Le capteur 7 selon la présente invention par la standardisation de l'étalonnage en ligne durant la manipulation rend les mesures de turbidité fiables et reproductibles, permettant ainsi la comparaison parfaite de la turbidité des cultures menées en parallèle. De plus, le coût est réduit et il est possible d'adapter d'autres capteurs (comme celui de bioluminescence) sur le porte-capteur.

Le capteur 7 à défilement, décrit ci-dessus, intégré dans la plate-forme 1 permet d'avoir un appareillage efficace dans le domaine des micro-cultures automatisées en parallèle. Outre la Génomique et la Protéomique, de nombreuses autres applications sont possibles :
- Comparaison de souches, physiologie microbienne;
- Mise au point de milieux de culture;
- Étude des effets simultanés des facteurs multiparamétriques environnementaux (microbiologie prédictive). Cas de souches délicates à cultiver (latence et temps de génération élevés);
- Optimisation des procédés de culture, scale-up;
- Étude des biofilms;
- Dépollution (étude de la décantation, enrichissement de souches, cultures mixtes);
- Screening de souches;
- Criblage de composés d'intérêt pharmaceutique;
- Biotransformations; et
- Thérapie cellulaire, état physiologique.

Les secteurs d'application couvrent de nombreux domaines :
- Agroalimentaire;
- Industrie pharmaceutique;
- Microbiologie clinique;
- Environnement (biofilms, polluants), dépollution et traitements;
- Laboratoires et industries des fermentations; et
- Recherche.

A titre d'exemple, on décrit plus bas une application majeure dans le domaine de la biotechnologie, soit l'étude des biofilms.

Une des causes essentielles des maladies nosocomiales (10 000 morts par an en France) est la formation de biofilms bactériens et de levures sur les cathéters ou divers implants médicaux ou chirurgicaux. Pour mettre au point des stratégies thérapeutiques anti-biofilms, il est nécessaire, d'une part, d'étudier le développement de ces biofilms et de trouver des drogues susceptibles de modifier leurs cinétiques de formation et, d'autre part, de caractériser les plastiques ou autres matières sur lesquels l'adhérence microbienne est diminuée voire supprimée. Les micro-fermenteurs avec la mesure en continu de la turbidité et la connaissance précise et reproductible des phases de croissance se prêtent parfaitement bien à une telle étude. En particulier, le capteur à défilement peut jouer un rôle essentiel.

Prenons l'exemple simplifié suivant selon lequel on veut observer les propriétés antiadhérentes de plastiques de diverse nature, par exemple du nylon, téflon, PVC, polycarbonate, etc. On introduit dans les tubes des micro-fermenteurs des petites languettes de différents plastiques, de telle façon que leurs faces soient perpendiculaires au faisceau lumineux du capteur de turbidité. On ensemence la culture avec une souche microbienne hyperadhérente. La croissance s'effectue et la souche adhère sur les différentes languettes avec une cinétique particulière liée à la nature du plastique. Le capteur à défilement est programmé pour se positionner précisément, à des intervalles de temps programmés, devant les différentes languettes. Les différences de turbidité mesurées permettent de déduire la sensibilité de ces plastiques à l'adhérence des cellules. Un autre essai complémentaire peut être d'ajouter des antibiotiques et d'observer la disparition des biofilms selon les matériaux et leurs épaisseurs. Pour tenir compte de l'adhérence sur le fermenteur lui-même, un dispositif peut consister à mesurer la turbibité à travers le fermenteur en y insérant 0, 1 ou 2 lamelles de verre. Les équations obtenues permettraient de connaître l'épaisseur du biofilm sur le fermenteur lui-même et la turbidité des cellules en suspension. Un autre dispositif pourrait consister à utiliser un tube présentant des zones avec plusieurs largeurs, donc avec des chemins optiques différents. Un autre moyen consisterait à traiter le fermenteur pour rendre sa surface interne non adhérente.

Dans le même ordre d'idée, il serait possible de déterminer les vitesses de décantation de cultures microbiennes et de mettre au point des systèmes d'enrichissement en mutants floculants, en vidangeant automatiquement les cultures à un certain taux et en rajoutant ensuite du milieu neuf (cultures cycliques).

Une autre façon d'analyser des cultures microbiennnes, pourrait consister à mesurer les turbidités à différentes longueurs d'onde et à traiter les équations obtenues, dont les coefficients sont fonction de la taille des cellules.

L'invention devrait s'avérer un outil important dans les domaines de la chimie et l'environnement, pour les mêmes raisons de diminution des coûts, de l'augmentation de la puissance analytique par l'utilisation des multi-capteurs, de l'augmentation de la fiabilité des mesures, de l'automatisation des mesures et du traitement informatique en ligne.

La présente plate-forme de cultures automatisée utilise aujourd'hui des réacteurs en verre qu'on doit conditionner et autoclaver avant chaque nouvel usage. De même, l'introduction de milieu stérile dans les réacteurs se fait sous une hotte bactériologique. Ces contraintes peuvent être perçues comme des inconvénients dans le sens d'une perte de temps entre chaque cycle de culture, alors que ces cycles de culture sont totalement automatisés. On pourrait ainsi concevoir un système de stérilisation en ligne des réacteurs, couplé à une introduction automatisée de milieu de culture. D'autre part, on peut envisager la mise au point de réacteurs stériles en polycarbonate, prêts à l'emploi et à usage unique. L'une ou l'autre de ces deux solutions devraient renforcer la dimension automatisée et à terme robotisée de la technologie proposée.

### Contrôle et régulation indépendante de la température pour chaque culture

La régulation de la température de croissance est un paramètre essentiel dans la conduite d'une culture microbienne. Le plate-forme 1 selon l'invention comporte donc un système de régulation de la température 17 (voir à la Figure 8). Cette régulation pour tous les micro-fermenteurs 3 de la batterie peut préférentiellement être une régulation de la température par effet Peltier, indépendante et programmable pour chaque micro-fermenteur 3 (plage de -5°C à 80°C).

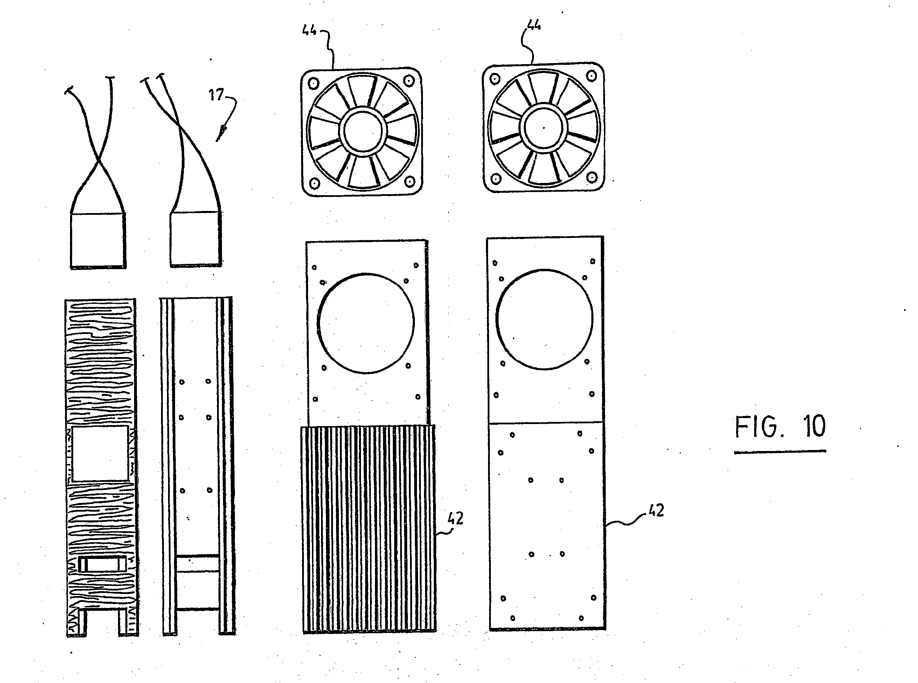

Le système à effet Peltier 17 qui est illustré aux Figures 8, 9A, 9B et 10 comprend des dissipateurs thermiques 42 munis de ventilateurs 44.

Le contrôle de la température est assuré par des régulateurs PID (Proportionnel Intégral Dérivé) autonomes et indépendants, couplés à des modules à effet Peltier. Les températures de consigne (0 à 65 °C) et celles mesurées par des sondes Pt100 sont transmises via une liaison RS-485 entre les modules thermiques et l'ordinateur.

La possibilité de programmation des températures de culture est un facteur essentiel dans l'optimisation des procédés bactériologiques. La possibilité de fixer automatiquement la température, ou de réaliser des incréments de température, permet d'optimiser la production de protéines sous forme soluble, en effectuant par exemple les cultures à basse température. La programmation indépendante de ce paramètre pour chaque réacteur, permet selon la souche bactérienne à cultiver, ou selon la séquence spécifique du gène à exprimer, de choisir la température la plus favorable à la croissance ou à l'expression de protéines recombinantes.

### Automatisation des opérations d'injection et de prélèvement d'échantillons en cours de procédés

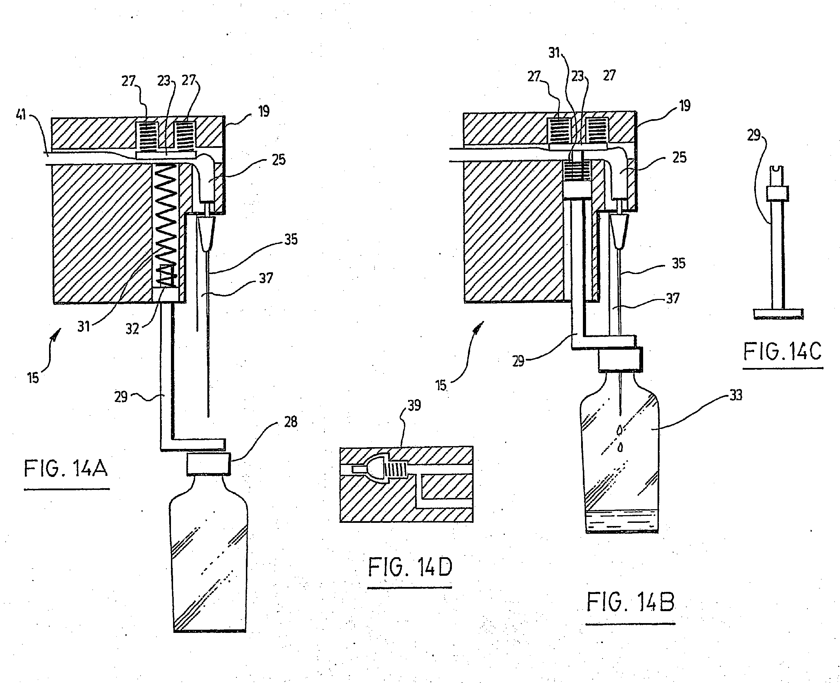

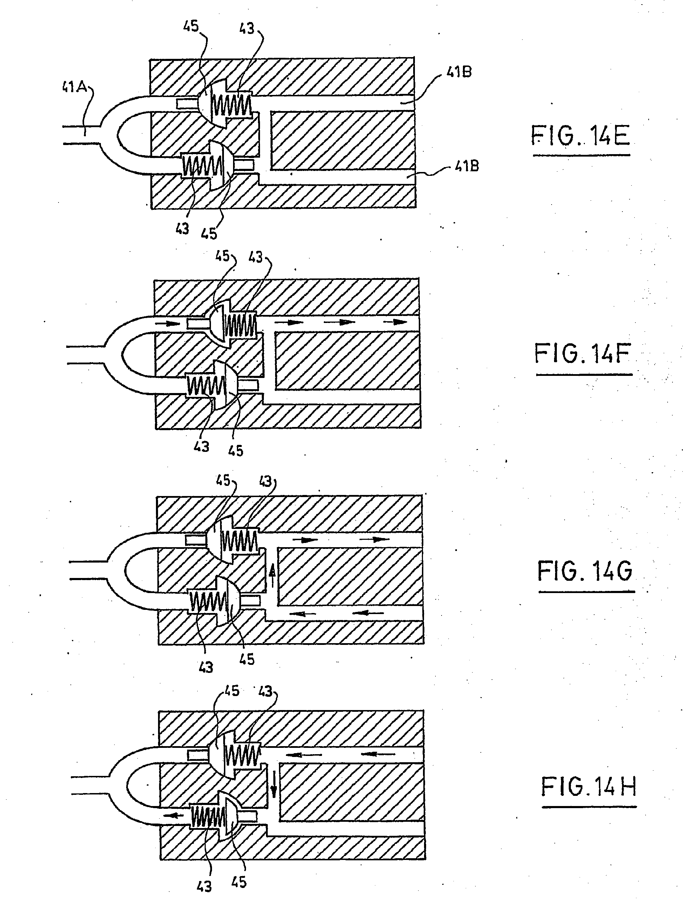

Tel que montré aux Figures 14A et 14B, un automate injecteur/échantillonneur 15 est intégré à la plate-forme 1 de cultures en micro-fermenteurs 3. Il permet, en fonction d'une valeur de la turbidité mesurée en temps réel ou du temps ou de tout autre paramètre mesuré dans le réacteur, et prédéfinis par un opérateur, d'injecter automatiquement un ou plusieurs produits (substrats, inducteur). L'automate injecteur/échantillonneur 15 peut fonctionner de façon synchronisé avec le capteur 7 de mesure de turbidité en ligne, et assure l'introduction automatique de l'inducteur à la phase de culture correspondant à la valeur de turbidité programmée par l'utilisateur. En outre, dans le cas où il est nécessaire de suivre des cinétiques d'expression de protéines, cet automate 15 permet de faire des prélèvements de volumes variables sur toutes les cultures en cours et de les stocker à 4°C. Chaque opération est supervisée par le programme informatique gérant l'ensemble des tâches de la plate-forme.

Tel qu'expliqué précédemment, la plate-forme 1 comporte un support régulé en température grâce à des modules à effet Peltier, capables de maintenir une température de consigne de 4°C. Ce support accepte des blocs amovibles (350 x 25 mm par exemple) percés de 20 à 24 trous pouvant recevoir des petits flacons de 12 x 32 mm, d'une capacité de 0,2 mL à 2 mL maximum. Des blocs adaptés à des flacons de tailles différentes peuvent être intégrés sur le support. Dans ce cas, les blocs comportent des éléments d'identification permettant au système de pilotage du support de s'adapter automatiquement.

Une zone de stockage surplombe une des extrémités du support et protège des porte-aiguilles amovibles 19 dont le nombre est fonction du nombre de réacteurs utilisés. Cette zone de stockage est équipée de petites résistances chauffantes chargées de stériliser l'extrémité des aiguilles et d'un collecteur pour drainer des projections éventuelles de liquide.

Les porte-aiguilles 19 sont placés sur des modules monte-baisse motorisés fixés sur un guide mobile circulant le long de deux rails parallèles. Ce guide mobile est solidaire d'une courroie crantée entraînée par un moteur pas à pas sous contrôle informatique. On comprendra que le guide mobile peut être solidaire du porte-capteur 9 décrit précédemment. Bien entendu, il est possible d'avoir des guides mobiles pour les porte-aiguilles 19 qui soient préférentiellement indépendants du porte-capteur 9 contenant le capteur de turbidité.

Les porte-aiguilles 19 sont amovibles et installés sur l'automate en fonction du nombre de voies utilisées. À charge du contrôle informatique d'identifier nombre et position des porte-aiguilles 19, ainsi que le type de blocs pour flacons 33 et de proposer à l'utilisateur un choix restreint et adapté d'actions. Pour élargir les possibilités d'utilisation, il peut être intéressant d'utiliser deux (2) systèmes de déplacement capables de gérer chacun quatre (4) porte-aiguilles.

Tel que montré à la Figure 14A, un porte-aiguille 19 est composé de 4 éléments principaux :
- Un galet presseur 23 écrasant un tuyau souple 25 par le biais de deux ressorts 27;
- Un piston mobile 29 à rappel par ressort 31 et dont la partie inférieure est constituée d'une tête permettant le centrage et le maintien du col du flacon 33 pendant l'opération, et d'une encoche 32 à l'extrémité supérieure permettant de soulever le mécanisme presseur 23 et de laisser le fluide circuler;
- Une aiguille 35 renforcée reliée au tuyau souple 25; et
- Un canal de purge 37 dont l'une des extrémités, parallèle à l'aiguille, est utilisée pour transmettre une «information pneumatique» récupérée par le purgeur 39.

Le purgeur 39 est constitué d'un circuit à 4 voies dont 2 sont reliées au réacteur par un tube capillaire 41A et équipées chacune d'un ressort de contre-pression 43 et d'une valve en caoutchouc 45 placés en opposition. Les deux autres voies 41 B sont reliées au porte-aiguille 19 par deux tubes capillaires. Le volume mort est donc simplement fonction de la distance entre le réacteur et le purgeur et du diamètre intérieur du capillaire utilisé pour les connecter. Le purgeur 39 ne nécessite aucune commande supplémentaire. Il est stérilisable.

Des flacons 33 pressurisés ou sous vide permettent d'éviter d'avoir recours à des électrovannes ou des pompes. Il est aisé de trouver dans le commerce des flacons de faible volume stériles et sous vide. Pour les transferts sous pression, on injectera le soluté et le volume d'air nécessaire à la compression avec une aiguille fine à travers un bouchon spécifique à épaisseur variable, dans un flacon déjà serti et stérile.

Une autre solution serait l'utilisation d'un tube capsulé dont le fond serait remplacé par un bouchon étanche et résistant à une piqûre d'aiguille. La surpression dans le micro-fermenteur 3 étant très faible, la pression interne nécessaire dans le flacon 33 ne devrait pas excéder 0,5 à 1 bar.

Les portes-aiguilles 19 étant solidaires d'un même guide mobile, l'automate ne peut effectuer qu'une action à la fois. Le programme de contrôle doit lui indiquer avec quelle aiguille et sur quel flacon il doit opérer, et mémoriser les événements en attente dans une pile « premier entré - premier sorti ». Considérant que la durée d'un cycle serait de 15 secondes au maximum, le temps d'attente entrera demande d'opération et son exécution serait de 2 minutes maximum (cas de 8 micro-fermenteurs), ce qui est largement suffisant à l'échelle d'une culture bactérienne.

Un cycle standard comporte les actions suivantes :
- Le guide mobile positionne précisément les modules porte-aiguilles 19 à l'aplomb du flacon 33 choisi. Le module monte-baisse entraîne le porte-aiguille 19 correspondant vers le flacon 33 pour les opérations de transfert; et
- L'aiguille 35 traverse le bouchon en caoutchouc du flacon 33 et le porte-aiguille 19 atteint sa position basse prédéfinie. Au contact avec le col du flacon 33 le piston 29 remonte et libère momentanément le galet presseur 23 à l'intérieur du porte-aiguille 19. L'extrémité du canal de purge 37 est bouchée par un contact ferme avec le bouchon 28 du flacon 33 (mais sans le percer).

Si le flacon 33 est pressurisé et contient une solution à transférer dans le réacteur :
- Le transfert du liquide s'effectue à travers la sortie (OUT) du purgeur 39 car la voie IN ne peut que se fermer sous la pression (voir à la Figure 14H); et
- Le volume de gaz comprimé en excès suffit à purger le tube capillaire.

Si le flacon 33 est en dépression :
- Le liquide est aspiré uniquement par l'entrée supérieure (IN) vers le flacon 33 car les 2 autres voies sont obturées (voir Figure 14F);
- Au bout d'un certain temps, le ressort de contre-pression 43 égale la dépression et le transfert s'interrompt. Mais il existe toujours à l'intérieur du flacon 33 une dépression résiduelle, identique à la force du ressort (voir Figure 14G);
- Le porte-aiguille 19 remonte d'un cran (ou lentement) et libère le canal de purge 37 (aidé en cela par le ressort de rappel 31 du piston 29 qui repousse le flacon 33) sans pour autant totalement dégager l'aiguille 35. L'air (cette voie devra éventuellement comporter un miro-filtre stérilisant) pénètre dans le canal 37 et transvase le liquide encore contenu dans le tube capillaire entre le purgeur 39 et le flacon 33;
- Le piston 29 repousse en fin de course le flacon 33 et le galet presseur 23 revient en position;
- Le module monte-baisse ramène en position haute le porte-aiguille 19 (voir Figure 14A); et
- Le guide mobile se repositionne dans sa zone d'attente et les aiguilles sont stérilisées par la chaleur des mini-résistances.

L'automate injecteur/échantillonneur 15 apporte un concept nouveau parfaitement intégré à la batterie de micro-fermenteurs 3.

L'automate 15 permet l'asservissement des actions grâce à des tubes d'injection et de prélèvement en pression ou dépression. Les volumes des solutions injectées et des échantillons sont prédéterminés. Le dispositif ne nécessite pas d'autres éléments extérieurs, comme des électrovannes, des mises en pression des fermenteurs, des pompes, etc.

L'automate 15 n'a besoin que d'une seule ligne pour les injections et les prélèvements, il offre les avantages suivants : compacité; minimum de ligne de canalisation (volume mort); grande ergonomie; possibilité informatique de gérer l'emplacement de chaque tube (traçabilité); pas de risque de contamination croisée.

L'utilisation de tubes en surpression et tubes en dépression qui sont à usage unique simplifie et sécurise les manipulations. Au niveau du laboratoire, la mise en pression et dépression peut se faire simplement à l'aide d'une seringue stérile sous une hotte à flux laminaire, en utilisant de préférence des bouchons à double épaisseur. D'autres dispositifs peuvent être utilisés.

L'ouverture des canalisations s'effectue au niveau du porte-aiguille 19 par un dispositif mécanique, la came (galet presseur 23), simple, robuste et peu coûteux, dont le mouvement est fonction de la position de l'aiguille 35 dans le flacon 33. Le purgeur 39 et le canal de purge 37 permettent de vider complètement, sans commande supplémentaire, les canalisations après les opérations de prélèvement.

Une autre variante, plus simple au point de vue mécanique mais moins performante pour le procédé, consiste à utiliser un porte-aiguille dépourvu du purgeur et du canal de purge. Cette variante nécessite cependant, avant chaque opération de prélèvement, une purge qui s'effectue par le déplacement du porte-aiguille vers un tube ou flacon poubelle.

Une troisième variante de porte-aiguille est envisageable : cet autre dispositif consiste en une double ou triple aiguille (une reliée au fermenteur comme précédemment, une amenant un gaz sous pression, une branchée sur le vide) pour des opérations plus complexes (par exemple des alimentations ou des prélèvements de volumes importants).

L'automate 15, intégré à la plate-forme 1 de cultures en batteries de micro-réacteurs 3, apporte un nouveau concept d'introduction de solutions et de prélèvements d'échantillons, permettant de mener en parallèle et de façon totalement automatisée des cultures depuis l'introduction des pré-cultures jusqu'au stockage final.

L'introduction de liquides dans les micro-fermenteurs peut s'effectuer par différents moyens conventionnels :
- Des flacons en surpression, contenant des milieux de culture ou des solutions quelconques, sont reliés aux fermenteurs (ceux-ci sont pratiquement à la pression atmosphérique sauf si les sorties d'effluents sont obturées) par des canalisations (en mannifolds). Des électrovannes Tout Ou Rien (TOR) à écrasement ou proportionnelles (stérilisables) sont pilotées par l'ordinateur. L'ouverture de celles-ci permet la circulation des liquides grâce au gradient de pression de l'amont vers l'aval. Les débits peuvent être réglés par des débitmètres ou grâce à des capillaires exerçant des pertes de charge variables (tube téflon de 0,5 mm par exemple). Pour une pression amont constante, les -débits sont remarquablement constants. Ils sont une fonction simple des longueurs de capillaire; et
- Des seringues ou une pompe contenant la solution à introduire sont reliées à un ou plusieurs fermenteurs à l'aide de manifolds. Les pousse-seringues pilotables permettent une alimentation variable selon les besoins de la culture. L'avantage des seringues est d'avoir des débits constants, quelle que soit la viscosité des milieux.

Ces dispostifs peuvent permettre l'introduction de solutions, par exemple en ouvrant une électrovanne ou en actionnant le pousse-seringue, ou le prélèvement d'échantillons, par exemple en bloquant la sortie des effluents gazeux par fermeture d'une électrovanne (d'ou mise en surpression du fermenteur) et en autorisant le passage du prélèvement par ouverture d'une autre électrovanne (on peut aussi utiliser une électrovanne à pincement à deux voies, une normalement fermée au repos (purge), une normalement ouverte (évent)).

Cependant, ces dispositifs sont plus difficilement adaptés à la plate-forme pour toute une série de raisons :
- Encombrement;
- Coût; et
- Complexité de montage.

L'automate injecteur/échantillonneur 15 apporte des avantages aussi bien au niveau des performances de l'appareillage que dans la conduite automatisée des procédés.

Un des avantages de la présente invention se situe au niveau de l'asservissement des actions grâce à des tubes en pression ou dépression. Ainsi, le fait de disposer de tubes en pression ou dépression de volumes variables simplifie énormément les procédures de culture, par exemple:
- Des tubes de 0,5 mL à 2 mL ou plus en surpression, à 4°C, contiennent chacun les différentes pré-cultures en phase exponentielle. Au moment de l'injection, les cultures (60 mL) sont ensemencées au 1/20 (ce qui est classique comme dilution);
- Des tubes de 2 mL contiennent 0,5 mL à 2 mL ou plus de solution d'inducteur qui sont introduits à la DO convenable; et
- Des tubes de 0,5 mL à 2 mL en dépression vont servir à recevoir les prélèvements. Un mL est amplement suffisant pour effectuer les analyses courantes.

Le fait de n'avoir qu'une ligne utile à la fois pour les injections et les prélèvements procure de nombreux avantages:
- Une plus grande ergonomie, pour éviter des lignes multiples souvent difficiles à identifier, d'où une plus grande facilité opérationnelle et moins de risque de contamination;
- Le volume de cette ligne est minimalisé pour éviter des volumes morts et des pertes de culture, ce qui n'est pas le cas lorsqu'on utilise une pompe; et
- Pas de contamination croisée, grâce au principe de circulation des liquides et à la stérilisation des aiguilles.

Les tubes en surpression et tubes en dépression permettent le développement de produits consommables (tubes en verre ou en plastique obturés par un septum, mannifolds de lignes d'alimentation avec des raccords éprouvés, etc.) à usage unique, spécifiques de la présente technologie, pouvant contenir des solutions prêtes à l'emploi : inducteurs, antibiotiques, milieux particuliers, etc., d'où des avantages économiques et pratiques.

Par ailleurs, le système de vidange automatique des canalisations apporte encore une plus grande simplicité dans le fonctionnement.

La conduite automatique d'une culture complète et de bonne qualité implique :
- Des pré-cultures en phase exponentielle;
- Un suivi de la culture en mesurant l'évolution des paramètres, en particulier la concentration cellulaire;
- Des actions, comme l'introduction de l'inducteur, au moment ou à la DO décidés;
- Des prélèvements pour évaluer la production en vue de la contrôler ou de l'optimiser; et
- Le stockage des cultures.

L'automate injecteur/échantillonneur 15, intégré aux micro-fermenteurs 3, contribue à remplir ces conditions, par exemple dans le cas de cultures recombinantes:
- Les pré-cultures obtenues en phase exponentielle de croissance sont stockées dans cet état sur les racks de l'automate à 4 °C, permettant ainsi un démarrage des cultures dans les réacteurs avec des phases de latence réduites;
- Elles sont injectées dans les micro-fermenteurs, selon le programme, par exemple à une heure donnée de la nuit, de façon à avoir le matin une culture en phase exponentielle;
- Les cultures croissent, souvent avec des cinétiques différentes. Lorsque, pour chaque culture, la DO d'induction est obtenue, l'inducteur est introduit automatiquement dans le micro-fermenteur en question;
- Des prélèvements sont effectués afin d'établir la cinétique de production de la protéine; et
- En fin de culture, après le nombre fixé d'heures d'induction, les cultures peuvent être directement refroidies dans le réacteur ou transférées dans des flacons de stockage.

L'automatisation des cultures est un facteur essentiel pour la standardisation et l'optimisation des procédures de culture. Elle contribue à un score élevé de bonnes cultures, tout en facilitant, par un enregistrement en ligne des opérations déclenchées et de leurs impacts sur la culture, l'interprétation des raisons d'une production moindre ou meilleure (mise en cause de la construction ou de la difficulté d'expression des gènes clonés : codons rares, protéines insolubles sous forme de corps d'inclusion; effets de constituants du milieu, de la température, etc.).

A terme, les tubes de prélèvement d'échantillons ou de fin de culture sont stockés sur les racks de l'automate qui pourront être pris par un robot pour pouvoir effectuer les étapes ultérieures des procédés de culture : centrifugation, cassage ou lyse des cellules, dosage des protéines, etc.

En ce qui concerne la traçabilité, les tubes peuvent être identifiés et ainsi permettre le suivi des diverses opérations.

L'automate injecteur/échantillonneur, décrit ci-dessus, intégré dans la plate-forme automatisée de micro-fermenteurs permet d'avoir un appareillage efficient dans le domaine des micro-cultures automatisées en parallèle.

Outre la Génomique et la Protéomique, de nombreuses autres applications sont possibles:
- Enrichissement, sélection, adaptation des microorganismes à des environnements particuliers;
- Comparaison de souches, physiologie microbienne;
- Mise au point de milieux de culture;
- Étude des effets simultanés des facteurs multiparamétriques environnementaux (microbiologie prédictive);
- Cas de souches délicates à cultiver (latence et temps de génération élevés);
- Optimisation des procédés de culture, scaling-up;
- Étude des biofilms;
- Dépollution (étude de la décantation, enrichissement de souches, cultures mixtes);
- Marquage de protéines (¹³C, ¹⁵N, sélénométhionine, eau lourde);
- Screening de souches;
- Criblage de composés d'intérêt pharmaceutique;
- Bio-transformations; et
- Thérapie cellulaire, état physiologique.

Les secteurs d'application couvrent de nombreux domaines :
- Agroalimentaire;
- Industrie pharmaceutique;
- Microbiologie clinique;
- Environnement (biofilms, polluants), dépollution et traitements;
- Laboratoires et industries des fermentations; et
- Recherche.

L'invention devrait s'avérer un outil important dans les domaines de la chimie et de l'environnement, pour des raisons de diminution des coûts et de l'automatisation des opérations d'injection et de prélèvement.

Certaines sociétés de fermenteurs de laboratoire de 1 à 20 litres utilisent le logiciel Labview® pour piloter les procédés de cultures. L'intégration de cet automate dans l'environnement Labview® selon la présente invention pourrait apporter un avantage significatif à ces appareillages.

### Implantation de micro-électrodes de pH et de pO₂, pour le suivi et la régulation de ces paramètres.

La platine des micro-fermenteurs 3 a été conçue pour recevoir des micro-électrodes de pH et de pO₂, pour la maîtrise de la régulation de ces paramètres et garantir des conditions de cultures optimales. Les différents gaz nécessaires circulent grâce à une surpression de 0,3 à 0,6 bar de l'amont vers l'aval. Un système d'électrovannes programmables permet de réaliser des mélanges gazeux air/O₂ optima pour obtenir des concentrations cellulaires très élevées (plus de 100 de DO pour la bactérie *E. coli*).

### Maintien du système de culture en surpression.

Afin de répondre à l'exigence de la stérilité des cultures bactériennes en cours, l'ensemble des cultures est maintenu en légère surpression. Pour une sécurité accrue, les effluents liquides ou gazeux sont canalisés et traités chimiquement.

### Procédés de cultures bactériologiques

Les potentialités d'utilisation de la plate-forme automatisée de cultures en Micro-Fermenteurs, ont été pleinement démontrées dans le domaine particulier et très actuel de l'expression de protéines recombinantes hétérologues, chez *E. coli* (protéines de *Mycobacterium -* projet Génopole IP). L'optimisation de procédés de culture (mise au point de milieux de culture spécifiques, optimisation des transferts d'oxygène dans les réacteurs, détermination des conditions optimales d'induction et de récolte) a permis d'atteindre des niveaux d'expression, en protéines solubles, très élevés. Ces résultats s'inscrivent dans l'objectif de la présente invention de proposer un outil automatisé de production, combiné à un savoir-faire, pour répondre aux nombreux programmes internationaux de génomique structurale.

### Gestion et supervision informatique des procédés

L'environnement électronique et informatique développé (interfaces électroniques de communication National Instruments et écriture des programmes sous logiciel Labview ®) permet, pour les huit cultures réalisées en parallèle, une conduite graphique et un suivi simplifié des procédés en cours, à toutes les étapes de leur déroulement. La programmation permet de gérer l'automatisation indépendante des huit (8) réacteurs, comme les phases d'introduction de solutés, de prélèvement d'échantillons, de réguler divers paramètres tel le pH et la pO₂, ou de refroidissement en fin de culture.

La plate-forme décrite ici correspond à un nouveau concept dans le domaine de la bactériologie, celui de pouvoir mener en parallèle et de façon totalement automatisée des cultures miniaturisées dans un système intégré sous forme de batteries de réacteurs.

### Les réacteurs de cultures

La section carrée des réacteurs de culture permet d'offrir une optique adaptée à la mesure en ligne de la turbidité. Le faible volume de 60 mL dans les conditions de culture qui ont été optimisées est suffisant pour les besoins de production en biomasse ou en protéines recombinantes en vue d'analyse cristallographiques ou fonctionnelle de ces protéines. Ce faible volume permet la réduction de l'encombrement des batteries. Ce volume de culture conduit à une utilisation moindre des milieux de culture parfois coûteux. L'utilisation de frittés poreux, pour la dispersion des gaz d'aération, et l'utilisation d'oxygène pur augmentent considérablement l'efficacité des transferts d'oxygène, et remplacent avantageusement les agitations mécaniques ou magnétiques. L'architecture de ces réacteurs permet l'implantation sur une platine, de micro-sondes de pH et de pO₂, reliées à des transmetteurs électroniques. Ces capteurs assurent la mesure de l'évolution de ces deux paramètres pendant la culture bactérienne. Une électrovanne assure le mélange de gaz d'aération (air et O₂) et peut maintenir un taux d'oxygène dissout défini par l'utilisateur. La régulation du pH est également prévue par ajout de solutions alcalines ou acides. Le programme informatique assure le suivi et la régulation P.I.D. de ces paramètres.

### Gestion et automatisation des procédés

On définit par automatisation des cultures miniaturisées, la possibilité de déclencher un événement ou une suite d'événements de façon programmée et sans intervention de l'utilisateur. L'originalité du système selon l'invention est que ce déclenchement d'événements est directement sous la dépendance d'une valeur de turbidité mesurée en ligne sur la culture. Cette valeur de turbidité est directement corrélée à la densité cellulaire dans le réacteur et constitue un paramètre essentiel de toutes les cultures. Le fait que cette mesure de turbidité puisse être réalisée de façon pratiquement continue, sur une plage très large de valeurs de densité cellulaire, sans aucune intervention de l'opérateur et perturbation sur les cultures, permet de déclencher, en parallèle sur toutes les cultures en cours, des fonctions diverses pratiquement instantanément, dès qu'une consigne particulière de turbidité a été atteinte. Cette automatisation est un gage de standardisation et de calibration des conditions sous lesquelles les cultures sont réalisées. Ces critères sont particulièrement importants dans des études de sélection de souches ou d'optimisation de procédés.

On rappelle ci-dessous les avantages spécifiques d'une réalisation de la présente invention sur les technologies de cultures en batteries commercialisées par les sociétés DASGIP et INFORS et précisons les avantages que seule la présente invention peut offrir :
- Miniaturisation des cellules de culture : les réacteurs développés ont un volume de 60 mL, et s'apparentent à des fermenteurs classiques du fait de la possibilité d'implanter des sondes de pH et de pO₂. Les technologies de DASGIP et d'INFORS proposent des réacteurs d'un volume minimum de 150 mL. Du fait du faible volume des réacteurs, les batteries selon l'invention présentent un encombrement réduit adapté aux contraintes d'espace dans les laboratoires;
- Garantie de la standardisation des cultures : le procédé est le seul sur le marché à proposer une mesure directe, séquentielle et en ligne de la turbidité de toutes les cultures en cours par une cellule unique de mesure à défilement. Il existe d'autres systèmes fondés sur des estimations indirectes de la concentration cellulaire, à travers la mesure de divers paramètres de culture (pH, consommation d'oxygène, potentiel rédox, concentrations de substrats, etc.). Ces mesures nécessitent, pour chaque culture, différents capteurs insérés dans le réacteur et sujets à des dérives en cours de fonctionnement. Dans le cas présent, le système de mesure, étant unique et localisé à l'extérieur du réacteur, offre une meilleure garantie de stabilité au niveau des réponses. Le présent système permet de connaître et de comparer en temps réel la phase de croissance de toutes les cultures réalisées en parallèle; et
- Automatisation des procédés : sur un plan général, l'automatisation d'un procédé de culture a pour objectif de limiter au maximum, voire d'exclure totalement, l'intervention de l'utilisateur sur le procédé en cours. Les avantages de cette technologie robotisée de culture sont multiples : standardisation des tâches réalisées par l'automate à travers des opérations mécaniques d'une très grande rapidité, stérilité, fiabilité et précision; possibilité d'appliquer au procédé une production à haut débit; possibilité de réaliser des cultures même si celles-ci nécessitent des plages horaires incompatibles avec la présence de l'utilisateur; intégration du procédé automatisé de culture dans une chaîne de traitement automatisé des produits de cette culture (centrifugation et traitement des culots ou des surnageants de culture, purification et analyse en ligne par d'autres automates).

La technologie de la plate-forme automatisée de cultures selon la présente invention est la seule à répondre, avec flexibilité, à l'ensemble de ces avantages. Les procédés de DASGIP et d'INFORS correspondent en fait à un système de cultures en réacteurs de faible volume (150 mL) en parallèle, mais ne sont pas totalement adaptés et conçus pour répondre à des objectifs d'automatisation.

Sur le plan plus spécifique de l'optimisation des cultures, l'automatisation permet d'optimiser les différentes étapes d'un procédé de culture :
- Les inoculum peuvent être arrêtés en phase exponentielle de croissance et conservés dans cet état, permettant un démarrage des cultures en minimisant la phase de latence;
- Au cours de la croissance des cellules, le contrôle automatisé des apports d'oxygène en fonction de la turbidité des cultures permet de travailler constamment à la concentration en O₂ la plus favorable et d'éviter, en cas d'anaérobiose partielle, la production d'acétate inhibiteur de la synthèse des protéines recombinantes ou en cas de concentration trop élevée d'O₂, la synthèse de protéines de stress (dont des protéases pouvant hydrolyser la protéine produite);

- Les changements automatisés de température, selon des séquences programmées se déclenchant en fonction de la mesure en ligne de la densité cellulaire ou de la valeur d'autres paramètres, permettent d'adapter et d'orienter le plus étroitement possible, les synthèses cellulaires dans le sens désiré par l'utilisateur. Il est ainsi possible de cultiver d'abord les cellules à la température autorisant un taux de croissance optimum (30 ou 37 °C) et obtenir rapidement de la biomasse, puis, afin de favoriser la synthèse de protéines solubles, de déclencher à une densité cellulaire prédéfinie, une baisse automatisée de la température (à 15°C ou moins si besoin) et une introduction de l'inducteur (IPTG par exemple) qui est contrôlée. En fin de phase d'induction, la température est automatiquement ramenée à la température choisie, par exemple 4°C, et permet ainsi de conserver la culture dans des conditions optimales d'attente de traitement (cas des cultures de nuit); et
- Les prises d'échantillons, permettant de suivre les cinétiques d'expression d'une protéine donnée, sont automatiquement réalisées par l'automate injecteur/échantillonneur.

L'automatisation des cultures est un facteur essentiel pour la standardisation et l'optimisation des procédures de culture. Elle contribue à un score élevé de bonnes cultures, tout en facilitant, par un enregistrement en ligne des opérations déclenchées et de leurs impacts sur la culture, l'interprétation des raisons d'une production moindre ou meilleure (mise en cause de la construction ou de la difficulté d'expression des gènes clonés : codons rares, protéines insolubles sous forme de corps d'inclusion; effets de constituants du milieu, de la température).

La présente invention permet la simplicité de la mise en oeuvre : la plate-forme est constituée d'une batterie compacte de huit (8) micro-réacteurs (extensible à douze (12) ou plus). Les micro-fermenteurs peu coûteux, d'entretien aisé, sont faciles à insérer dans les batteries et à raccorder de façon fiable aux diverses canalisations de circulation des gaz et des liquides. Les programmes graphiques de commandes sont conviviaux grâce à une présentation visuelle des procédés en cours, sur l'écran de l'ordinateur de commande. L'interface informatique a été développée pour une prise en main rapide du procédé par les utilisateurs. Elle permet de gérer la conduite des cultures, l'étalonnage précis des sondes et du capteur de turbidité à défilement et autorise, si nécessaire, la modification des paramètres de culture en fonction du comportement de la culture.

Lès diverses options ou procédés dérivés de la présente technologie de cultures automatisées, permettent d'ouvrir le champ à de nombreuses applications:
- Cultures automatisées de très faibles volumes pour la sélection de nouvelles molécules à usage thérapeutique, dirigées contre des souches bactériennes maintenues en croissance optimale ou dans des conditions prédéfinies par l'expérimentateur. Ces cultures seront réalisées dans des tubes aérés (2 à 5 mL), portés par des racks, avec mesure en ligne de la turbidité par le capteur à défilement et injection programmée de toute molécule en cours de culture. Aujourd'hui, la culture automatisée en micro-plaques agitées, très largement utilisées dans tous les projets bio-médicaux, est la seule disponible pour ce type de projet. Cependant la croissance des cultures utilisant cette technique est rapidement limitée par l'oxygène en raison d'un transfert passif des gaz vers la phase liquide. Cette technique est donc seulement compatible avec l'utilisation de milieux simples et ne permet pas de présumer du comportement des souches en milieux complexes utilisés en fermentation. De plus, les niveaux d'expression de protéines recombinantes en micro-plaque demeurent très faibles;
- Le concept de la plate-forme 1 est adaptable à des batteries équipées de fermenteurs ou de réacteurs de faibles volumes ou de plus grands volumes; et
- Cultures automatisées de volumes importants pour la production de protéines destinées aux études de génomique fonctionnelle. Cette technologie utilisera des réacteurs de culture qui sont déjà conçus et qui seront totalement compatibles avec les exigences de mesure en ligne de la turbidité et des différentes fonctions automatisées. Ces réacteurs sont cylindriques (de 125 à 500 mL) et présentent une excroissance adéquate pour la mesure de la turbidité. Le capteur à défilement de mesure en ligne de la turbidité, passe successivement devant les excroissances des réacteurs de la batterie, et se positionne au niveau des deux faces parallèles pour une lecture convenable. Dans la démarche d'études systématiques des protéines codées par les gènes de génomes séquencés, de nombreuses protéines ne s'expriment que très faiblement. Ces protéines sont toxiques pour les souches bactériennes qui hébergent les gènes correspondants, ou présentent des caractéristiques biochimiques et fonctionnelles (protéines membranaires) peu favorables à leur expression sous forme soluble. L'augmentation des volumes de culture, couplée aux avantages de l'automatisation, devrait permettre de compenser ces faibles niveaux d'expression et de répondre ainsi aux attentes des études de fonctionnalité de ces protéines.

Cette technologie pourra également s'appliquer à l'optimisation de procédés devant être transférés à des échelles importantes de production :
- Cultures en réacteurs miniaturisés pour le marquage moléculaire des protéines, en vue d'études RMN et cristallographiques. L'utilisation de volumes réduits diminue le coût des milieux marqués très onéreux;
- Cultures par exemple en batch, fed batch, en turbidostat, cultures cycliques, cultures continues en chemostat, pour des études d'évolution ou de sélection de souches et des études physiologiques. Le faible volume des cultures (60 mL) permet de réaliser des cultures de longue durée sans avoir à changer fréquemment les nourrices, changement fastidieux et pouvant être cause de contaminations;
- Implantation de la présente technologie de plate-forme automatisée de cultures en PSM (Poste de Sécurité Microbiologique) ou dans des locaux confinés de type P2 ou P3, avec suivi et surveillance des cultures à distance (Internet, e-mail d'alerte, etc); et
- Développement de produits consommables à usage unique, spécifiques de la présente technologie : réacteurs de culture en plastique, milieux de cultures prêts à l'emploi, etc.

L'appareillage décrit ci-dessus représente un concept original et évolutif dans le domaine des micro-cultures automatisées. Outre la Génomique et la Protéomique, de nombreuses autres applications sont possibles, moyennant des adaptations mineures (surtout au niveau des programmes de contrôle), par exemple :
- Enrichissement, sélection, adaptation des microorganismes à des environnements particuliers;
- Comparaison de souches, physiologie microbienne;
- Mise au point de milieux de culture;
- Étude des effets simultanés des facteurs multiparamétriques environnementaux (microbiologie prédictive);
- Cas de souches délicates à cultiver (latence et temps de génération élevés);
- Optimisation des procédés de culture, scaling-up;
- Etude des biofilms;
- Screening de souches;
- Criblage de composés d'intérêt pharmaceutique;
- Biotransformations;
- Marquage de protéines (¹³C, ¹⁵N, sélénométhionine, eau lourde); et
- Thérapie cellulaire.

Les secteurs d'application couvrent de nombreux domaines :
- Agroalimentaire;
- Industrie pharmaceutique;
- Microbiologie clinique;
- Environnement (biofilms, polluants), dépollution et traitements;
- Laboratoires et industries des fermentations; et
- Recherche.

Le tableau ci-dessous donne des résultats de validation du système selon un mode de réalisation préférentiel de la présente invention. On y montre le niveau d'expression d'un certain nombre de protéines, en utilisant les systèmes classiques de production de protéines ou en utilisant le système selon la présente invention.

| COMPARAISON DES QUANTITÉS DE PROTÉINES RECOMBINANTES SOLUBLES, OBTENUES EN FERBACH AGITÉ DE 1 LITRE ET EN MULTI MICRO FERMENTEURS DE 60 ML SELON LA PRÉSENTE INVENTION | | | |
|---|---|---|---|
| Protéines produites | Poids moléculaire Kda | Rendement (mg/affinité/protéine soluble purifiée) | |
| | | Fernbach de 1 litre LB média | Bioréacteur 60 ml selon la présente invention HD média |
| 1 | 19,8 | 15 | 100 |
| 2 | 23,5 | 60 | 60 |
| 3 | 28,7 | 20 | 20 |
| 4 | 25,3 | 20 | 22 |
| 5 | 55 | 47 | 40 |
| 6 | 21,56 | 68 | 67 |
| 7 | 27,17 | 29 | 31 |
| 8 | 17,6 | 29 | 35 |
| 9 | 21 | 4,6 | 8,4 |
| 10 | 24,2 | 40 | 50 |
| 11 | 24,2 | 78 | 94 |
| 12 | 26,4 | 37 | 52 |
| 13 | 27,7 | 38 | 66 |
| 14 | 25,19 | 40 | 66 |
| 15 | 24,31 | 42 | 56 |
| 16 | 20,9 | 150 | 150 |

## Revendications

1. Une plate-forme automatisée et robotisée comportant une batterie de réacteurs miniaturisés dont le volume utile est compris entre 2 mL et 500 mL contenant chacun une culture cellulaire, la plate-forme comprenant :
- un capteur externe pour mesurer au moins une propriété optique de chaque culture cellulaire contenue dans chaque réacteur miniaturisé ;
- un porte-capteur mobile en forme de fourche ou d'étrier, apte à recevoir le capteur externe, le porte- capteur comportant des moyens de déplacement du capteur pour déplacer le capteur externe d'un réacteur miniaturisé à un autre et pour permettre la mesure en temps réel de ladite au moins une propriété optique ;
- des moyens de contrôle et de traitement pour recevoir en temps réel des mesures de la propriété optique du capteur externe et pour contrôler en temps réel un déplacement du porte-capteur mobile, et
- un système de régulation de la température pour chaque réacteur miniaturisé.

2. La plate-forme selon la revendication 1, dans laquelle le capteur externe est un capteur de turbidité comprenant une diode émettrice et une diode réceptrice.

3. La plate-forme selon la revendication 1, comprenant en outre au moins un deuxième capteur externe monté sur le porte-capteur mobile.

4. La plate-forme selon la revendication 1, dans laquelle le capteur est un capteur d'absorbance ou de fluorescence ou de luminescence ou de phosphorescence ou de colorimétrie ou tout autre capteur mesurant un rayonnement électromagnétique.

5. La plate-forme selon l'une quelconque des revendications 1 à 4, dans laquelle les moyens de déplacement du capteur comprennent :
- soit au moins un chariot mobile monté sur au moins un rail linéaire, un moteur pas à pas, un système d'entraînement reliant le moteur au chariot ; ou
- soit un bras ou tout autre système permettant un mouvement circulaire ; et
- des moyens de contrôle reliés au moteur assurant le déplacement dudit chariot selon un mouvement linéaire ou circulaire.

6. La plate-forme selon l'une quelconque des revendications 1 à 5, comprenant en outre un système de prélèvement et d'injection monté sur un support mobile et relié préférentiellement à un système de déplacement indépendant ou solidaire du porte-capteur mobile.

7. La plate-forme selon l'une quelconque des revendications 1 à 6, dans laquelle le système de régulation de température est un système de régulation autonome par effet Peltier.

8. Méthode pour mesurer automatiquement au moins une propriété optique de cultures cellulaires contenues dans une batterie de réacteurs miniaturisés dont le volume utile est compris entre 2 mL et 500 mL, comprenant les étapes de :
- mesure automatique d'au moins une propriété optique d'une culture contenue dans l'un des réacteurs miniaturisés à l'aide d'un capteur externe ;
- déplacement robotisé du capteur externe vers un autre réacteur miniaturisé ; et
- mesure automatique d'au moins une propriété optique d'une culture contenue dans un autre réacteur miniaturisé à l'aide du capteur externe.

9. La méthode selon la revendication 8, comprenant en outre l'étape d'injection et/ou d'échantillonnage d'un réacteur miniaturisé en fonction de la valeur de la mesure de la propriété optique.

10. La plate-forme selon l'une quelconque des revendications 1 à 7 permettant de produire des cultures cellulaires.

11. La plate-forme selon l'une quelconque des revendications 1 à 7 permettant d'optimiser des procédés de cultures cellulaires.

12. La plate-forme selon les revendications 10 et/ou 11 permettant l'analyse de mécanismes d'expression des gènes.

13. La plate-forme selon la revendication 12 où les gènes sont des gènes impliqués dans des mécanismes d'adhérence cellulaire.

14. La plate-forme selon l'une quelconque des revendications 1 à 7 permettant l'étude de mécanismes physiques et physico-chimiques.

## Patentansprüche

1. Automatisierte und robotisierte Plattform, umfassend eine Batterie miniaturisierter Reaktoren, deren wirksames Volumen zwischen 2 ml und 500 ml inbegriffen ist und jeweils eine Zellkultur enthält, wobei die Plattform umfasst:
- einen externen Sensor, um wenigstens eine optische Eigenschaft jeder Zellkultur zu messen, die in jedem miniaturisierten Reaktor enthalten ist;
- einen mobilen Sensorträger in Gabel- oder Bügelform, der geeignet ist, den externen Sensor aufzunehmen, wobei der Sensorträger Versetzungsmittel des Sensors zum Versetzen des externen Sensors von einem miniaturisierten Reaktor zu einem anderen und um die Messung der genannten wenigstens einen optischen Eigenschaft in Echtzeit zu ermöglichen, umfasst;
- Kontroll- und Verarbeitungsmittel, um in Echtzeit Messungen der optischen Eigenschaft des externen Sensors zu empfangen und um in Echtzeit die Versetzung des mobilen Sensorträgers zu kontrollieren und
- ein Regulierungssystem der Temperatur für jeden miniaturisierten Reaktor.

2. Plattform gemäß Anspruch 1, bei der der externe Sensor ein Trübungssensor ist, umfassend eine Senderdiode und eine Empfängerdiode.

3. Plattform gemäß Anspruch 1, umfassend darüber hinaus wenigstens einen zweiten externen Sensor, der auf dem mobilen Sensorträger montiert ist.

4. Plattform gemäß Anspruch 1, bei der der Sensor ein Absorptivitäts- oder Fluoresenz- oder Lumineszenz- oder Phosphorenzenz- oder Kolorimetriesensor oder jeder andere Sensor ist, der eine elektromagnetische Strahlung misst.

5. Plattform gemäß irgendeinem der Ansprüche 1 bis 4, bei der die Versetzungsmittel des Sensors umfassen:
- entweder einen mobilen Wagen, der auf wenigstens einer linearen Schiene montiert ist, einen Schrittmotor, ein Antriebssystem, das den Motor an den Wagen anschließt; oder
- einen Arm oder jedes andere System, das eine kreisförmige Bewegung zulässt; und
- Kontrollmittel, die an den Motor angeschlossen sind und die Versetzung des genannten Wagens gemäß einer linearen oder kreisförmigen Bewegung erlauben.

6. Plattform gemäß irgendeinem der Ansprüche 1 bis 5, umfassend darüber hinaus ein Entnahme- und Einspritzsystem, das auf einen mobilen und bevorzugt an ein unabhängiges oder mit dem mobilen Sensorträger fest verbundenes Versetzungssystem angeschlossen ist.

7. Plattform gemäß irgendeinem der Ansprüche 1 bis 6, bei der das Regulierungssystem der Temperatur ein autonomes Regulierungssystem per Peltier-Effekt ist.

8. Verfahren zur automatischen Messung wenigstens einer optischen Eigenschaft von Zellkulturen, die in einer Batterie miniaturisierter Reaktoren enthalten sind, deren wirksames Volumen zwischen 2 ml und 500 ml inbegriffen ist, umfassend die Stufen:
- der automatischen Messung wenigstens einer optischen Eigenschaft einer in einem der miniaturisierten Reaktoren enthaltenen Kulturen mithilfe eines externen Sensors;
- der robotisierten Versetzung des externen Sensors zu einem anderen miniaturisierten Reaktor; und
- der automatischen Messung wenigstens einer optischen Eigenschaft einer in einem anderen miniaturisierten Reaktor enthaltenen Zellkultur mithilfe des externen Sensors.

9. Verfahren gemäß Anspruch 8, umfassend darüber hinaus die Einspritz- und / oder Probennahmenstufe eines miniaturisierten Reaktors in Abhängigkeit von dem Wert der Messung der optischen Eigenschaft.

10. Plattform gemäß Anspruch 1 bis 7, die die Herstellung von Zellkulturen zulässt.

11. Plattform gemäß irgendeinem der Ansprüche 1 bis 7, die die Optimierung der Verfahren von Zellkulturen zulässt.

12. Plattform gemäß Ansprüche 10 und / oder 11, die die Analyse von Expressionsmechanismen der Gene zulässt.

13. Plattform gemäß Anspruch 12, bei der die Gene Gene sind, die in Mechanismen zellulärer Adhäsion impliziert sind.

14. Plattform gemäß irgendeinem der Ansprüche 1 bis 7, die die Studie von physikalischen oder physikalisch-chemischen Mechanismen zulässt.

## Claims

1. An automated and robotized platform comprising a battery of miniaturized reactors the useful volume of which is in the range 2 mL to 500 mL, each containing a cell culture, the platform comprising:
• an external sensor for measuring at least one optical property of each cell culture contained in each miniaturized reactor;
• a mobile sensor holder in the form of a fork or stirrup, capable of receiving the external sensor, the sensor holder comprising means for displacing the sensor in order to displace the external sensor from one miniaturized reactor to another and to allow said at least one optical property to be measured in real time;
• processing and control means for receiving the measurements of the optical property from the external sensor in real time and for controlling a displacement of the mobile sensor holder in real time; and
• a temperature regulating system for each miniaturized reactor.

2. The platform as claimed in claim 1, in which the external sensor is a turbidity sensor comprising an emitting diode and a receiving diode.

3. The platform as claimed in claim 1, further comprising at least one second external sensor mounted on the mobile sensor holder.

4. The platform as claimed in claim 1, in which the sensor is an absorbance or fluorescence or luminescence or phosphorescence or colorimetric sensor or any other sensor measuring an electromagnetic radiation.

5. The platform as claimed in any one of claims 1 to 4, in which the sensor displacement means comprise:
• either at least one mobile carriage mounted on at least one linear track, a stepper motor, a drive system connecting the motor to the carriage; or
• at least one arm or any other system for providing a circular movement; and
• control means connected to the motor to provide the displacement of said carriage in a linear or circular movement.

6. The platform as claimed in any one of claims 1 to 5, further comprising a removal and injection system mounted on a mobile support and preferably connected to a displacement system which is independent from or integral with the mobile sensor holder.

7. The platform as claimed in any one of claims 1 to 6, in which the temperature regulating system is an autonomous Peltier effect regulating system.

8. A method for automatically measuring at least one optical property of cell cultures contained in a battery of miniaturized reactors the useful volume of which is in the range 2 mL to 500 mL, comprising the steps of:
• automatically measuring at least one optical property of a culture contained in one of the miniaturized reactors with the aid of an external sensor;
• robotically displacing the external sensor towards another miniaturized reactor; and
• automatically measuring at least one optical property of a culture contained in another miniaturized reactor with the aid of the external sensor.

9. The method as claimed in claim 8, further comprising the step of injecting and/or sampling a miniaturized reactor as a function of the value of the measurement of the optical property.

10. The platform as claimed in any one of claims 1 to 7, suitable for the production of cell cultures.

11. The platform as claimed in any one of claims 1 to 7, suitable for optimizing cell culture processes.

12. The platform as claimed in claim 10 and/or 11, suitable for analysing gene expression mechanisms.

13. The platform as claimed in claim 12, in which the genes are genes involved in cellular adhesion mechanisms.

14. The platform as claimed in any one of claims 1 to 7, for the study of physical and physico-chemical mechanisms.
